(19) Europäisches Patentamt
European Patent Office
Office européen des brevets

(11) EP 2 931 728 B1

(12) **EUROPEAN PATENT SPECIFICATION**

(45) Date of publication and mention of the grant of the patent:
**19.02.2020 Bulletin 2020/08**

(21) Application number: **13821500.9**

(22) Date of filing: **13.12.2013**

(51) Int Cl.:
***C07D 487/22*** *(2006.01)* ***A61K 31/41*** *(2006.01)*
***A61P 3/00*** *(2006.01)* ***A61P 9/00*** *(2006.01)*
***A61P 17/00*** *(2006.01)* ***A61P 19/00*** *(2006.01)*
***A61P 31/00*** *(2006.01)* ***A61P 35/00*** *(2006.01)*
***A61P 43/00*** *(2006.01)*

(86) International application number:
**PCT/GB2013/053285**

(87) International publication number:
**WO 2014/091241 (19.06.2014 Gazette 2014/25)**

(54) **CHLORIN DERIVATIVE USEFUL IN PHOTODYNAMIC THERAPY AND DIAGNOSIS**
CHLORINDERIVATE FÜR FOTODYNAMISCHE THERAPIE UND DIAGNOSE
DÉRIVÉ DE CHLORIN UTILE EN THERAPIE PHOTODYNAMIQUE ET DIAGNOSTIC

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **14.12.2012 GB 201222544**
**23.05.2013 GB 201309337**

(43) Date of publication of application:
**21.10.2015 Bulletin 2015/43**

(73) Proprietor: **RMW Cho Group Limited**
**Hong Kong (CN)**

(72) Inventor: **CHO, Honsue**
**Melbourne, Victoria 3102 (AU)**

(74) Representative: **Johnson, Stephen William et al**
**Venner Shipley LLP**
**Byron House**
**Cambridge Business Park**
**Cowley Road**
**Cambridge CB4 0WZ (GB)**

(56) References cited:
**WO-A1-2009/040411** **WO-A2-03/028629**
**CN-A- 1 687 089** **GB-A- 2 389 531**

- **K.C. WONG ET AL.: "The influence of calcium on the cardiotropic actions of rhodochlorin and ouabain", JOURNAL OF PHARMACOLOGY AND EXPERIMENTAL THERAPEUTICS, vol. 155, no. 1, 1967, - 1 January 1967 (1967-01-01), pages 50-57, XP008167388,**
- **BIN LI ET AL: "Chlorophyllin e4 is a novel photosensitizer against human bladder cancer cells", ONCOLOGY REPORTS, vol. 27, no. 5, 26 January 2012 (2012-01-26), pages 1455-1460, XP55101722, GR ISSN: 1021-335X, DOI: 10.3892/or.2012.1656**
- **M. J. Hendrickson ET AL: "Radial Paper Chromatography...Application to Analysis of Mixtures of Chlorophyll Derivatives", Analytical Chemistry, vol. 29, no. 12, 1 December 1957 (1957-12-01), pages 1810-1815, XP055353908, US ISSN: 0003-2700, DOI: 10.1021/ac60132a043**
- **HENDRICKSON ET AL: "1310. SOME PHYSICAL AND CHEMICAL CHARAC. TERISTICS OF RHCDOCHLORIN, A NEW CARDJO. TROPIC COMPOUND", FEDERATION PROCEEDINGS, FEDERATION OF AMERICAN SOCIETIES FOR EXPERIMENTAL BIOLOGY, US, vol. 16, 1 January 1957 (1957-01-01), page 306, XP008183639, ISSN: 0014-9446**
- **R.B. WOODWARD: "Totalsynthese des Chlorophylls", ANGEWANDTE CHEMIE, vol. 72, no. 18, 1960, pages 651-662,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



(Cont. next page)

- **E. S. SIMAREMARE ET AL.: "Photophysical and physiochemical properties of Cu(II)chlorin e4 and Cu(II)chlorin e6 as a lead compound of photosensitizer for PDT", INDONESIAN J. PHARM, vol. 26, no. 1, 2015, pages 29-36,**
- **D. L. Murphy et al, Poster "Real-time fluorescent photography using Photosoft PDT and metabolic urine studies in prostate cancer patients", USANZ 2017**


Remarks:

The file contains technical information submitted after the application was filed and not included in this specification

## Description

### Technical field

The present invention relates to chlorin e4 sodium and a process for its preparation. Chlorin e4 sodium is suitable for use in photodynamic therapy, cytoluminescent therapy and photodynamic diagnosis, for example for treating or detecting a tumour. The present invention also relates to a pharmaceutical composition comprising chlorin e4 sodium, to the use of chlorin e4 sodium in the manufacture of a phototherapeutic or photodiagnostic agent, and to chlorin e4 sodium for use in photodynamic therapy, cytoluminescent therapy or photodynamic diagnosis, for example, for use in treating or detecting a tumour.

### Background art

Porphyrins and their derivatives are known photosensitive chemical compounds, which can absorb light photons and emit them at higher wavelengths. There are many applications for such unique properties and PDT (photodynamic therapy) is one of them.

During light activation, a molecule goes from the ground state ($S_0$) to a singlet excited state ($S_1$), ($S_0 \rightarrow S_1$ transition), and then falls back down to the ground state ($S_0$), ($S_1 \rightarrow S_0$ transition), emitting light at higher wavelengths in the form of fluorescence. There is another energy state known as the triplet state ($T_1$). The triplet state ($T_1$) cannot be efficiently populated by direct excitation. However, intersystem crossing $S_1 \rightarrow T_1$ occurs isoenergetically followed by deactivation to $T_1$. The $S_1 \rightarrow T_1$ change is formally spin-forbidden, but it is a downhill process and in some molecules intersystem crossing occurs so efficiently that fluorescence is quenched. Such compounds are useful for generating excited triplets by excitation transfer (a phenomenon known as sensitization) in molecules where the intersystem crossing is minimal.

Figure 1 shows a modified Jablonski diagram for a typical photosensitizer, outlining the principle photophysical processes of interest.

At the triplet state ($T_1$) molecules become very unstable and can react with molecular oxygen ($^3O_2$), splitting the latter into two singlet oxygens ($^1O_2$). Singlet oxygen ($^1O_2$) is known as a "scavenger" and can destroy abnormal biological cells and remove free radicals.

Such unique properties make many photosensitive molecules good photosensitizers for PDT.

Presently, there are two generations of photosensitizers for PDT. The first generation comprises hem porphyrins (blood derivatives), and the second for the most part are chlorophyll derivatives. The later compounds are known as chlorins and bacteriochlorins.

Chlorins show photophysical properties similar to those of the porphyrin systems, but have an enhanced, red-shifted Q band (670nm) which makes chlorin-containing systems better candidates for PDT. *Chlorophyll a,* the green photosynthetic pigment, is one prototype of the chlorin class of natural products. It or its derivatives can be extracted from certain *Spirulina Platensis* species without any contamination with *chlorophyll b,* thus avoiding a tedious chromatographic separation.

A water-soluble chlorophyll derivative, known as tri-sodium copper(II) chlorin e6, has been used in the treatment of various human ailments with no evidence of toxicity, skin sensitization or other serious side effects.

Wong et al (The Journal of Pharmacology and Experimental Therapeutics, 1967, vol. 155(1), pages 50-57) describe the cardiotropic actions of chlorin e4. Chlorin e4 was studied recently and was shown to display good photosensitive activity. It was indicated that chlorin e4 has a protective effect against indomethacin-induced gastric lesions in rats and TAA- or $CCl_4$-induced acute liver injuries in mice. It was therefore suggested that chlorin e4 may be a promising new drug candidate for anti-gastrelcosis and liver injury protection. WO 2009/040411 suggests the use of a chlorin e4 zinc complex in photodynamic therapy. Li et al (Oncology Reports, 2012, vol. 27, pages 1455-1460) describe chlorin e4 as a photosensitizer in the treatment of bladder cancer cell lines.

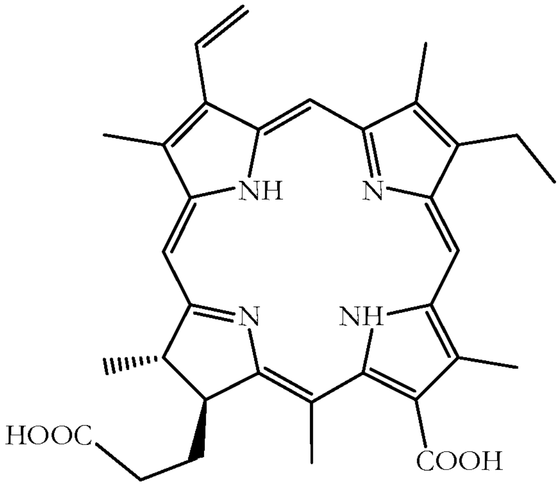

chlorin e4

However, there is an ongoing need for better photosensitizers. There is a need for compounds that have a high singlet oxygen quantum yield and for compounds that have a strong photosensitizing ability, preferably in organic and aqueous media. There is also a need for compounds that have a high fluorescence quantum yield.

## Summary of the invention

A first aspect of the present invention provides chlorin e4 sodium, having the structure:

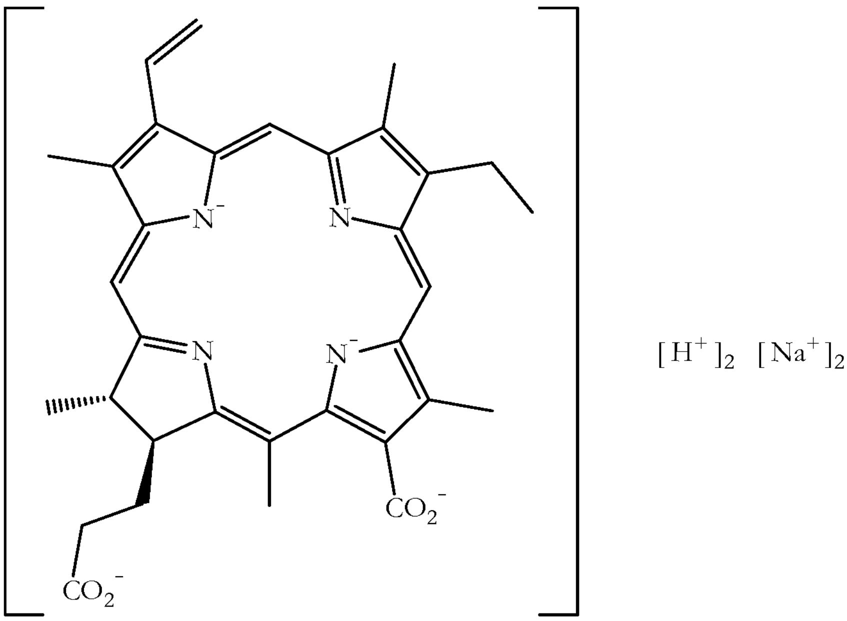

Preferably the chlorin e4 sodium has the structure:

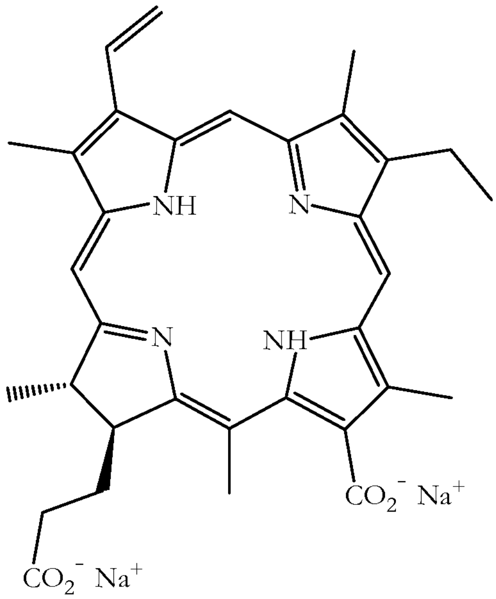

.

The skilled person will appreciate that the negative charges on the chlorin structures shown in the above representations are delocalized over the conjugated system.

Chlorin e4 sodium has two chiral centres. The chlorin e4 sodium of the present invention is preferably substantially enantiomerically pure, which means that the chlorin e4 sodium comprises less than 10% of other stereoisomers, preferably less than 5%, preferably less than 3%, preferably less than 2%, preferably less than 1%.

A second aspect of the present invention provides a process of preparing chlorin e4 sodium according to the first aspect of the present invention, comprising the step of reacting chlorin e4 with a sodium compound.

Preferably the sodium compound is sodium hydroxide, sodium carbonate, a carboxylic acid sodium salt (such as sodium stearate or sodium acetate), a sodium halide (such as sodium fluoride) or sodium hydride. Preferably the sodium compound is sodium hydroxide, a carboxylic acid sodium salt (such as sodium stearate or sodium acetate), a sodium halide (such as sodium fluoride) or sodium hydride. Preferably the sodium compound is sodium hydroxide or sodium carbonate. More preferably the sodium compound is sodium hydroxide.

Optionally chlorin e4 is reacted with the sodium compound in the presence of a base. Preferably the base is an amine base, preferably an aromatic amine base such as thiamine pyrophosphate or DMAP. If used, the base is preferably thiamine pyrophosphate.

Preferably chlorin e4, the sodium compound and the optional base are used in a molar ratio of 0.15-1 : 45-60 : 0-12.

Preferably chlorin e4 is reacted with the sodium compound in the presence of a solvent. Preferably the solvent is selected from an alcohol (such as methanol, ethanol, n-propanol or isopropanol), water, DMF, chloroform, DMSO, toluene or a mixture thereof. Preferably the solvent is selected from methanol, ethanol, water or a mixture thereof. Preferably the solvent is selected from methanol, water or a mixture thereof. Preferably the solvent is a mixture of methanol and water.

Preferably the reaction is carried out at a temperature of 60°C or less. Preferably the reaction is carried out at a temperature in the range of 25-60°C.

Preferably the reaction is carried out over 1 to 12 hours, preferably over 3 to 8 hours.

The chlorin e4 sodium obtained may be purified by filtering, for example through a 45μm Millipore filter. Alternatively or additionally, the chlorin e4 sodium obtained may be purified by chromatography, for example by flash chromatography on a silica gel column. The chlorin e4 sodium may be isolated by lyophilisation.

A fourth aspect of the present invention provides a pharmaceutical composition comprising chlorin e4 sodium according to the first aspect of the present invention and a pharmaceutically acceptable carrier or diluent.

Preferably the chlorin e4 sodium according to the first aspect of the present invention is suitable for use in medicine.

Preferably the chlorin e4 sodium according to the first aspect of the present invention and the pharmaceutical composition according to the fourth aspect of the present invention are suitable for use in photodynamic therapy or cytoluminescent therapy.

Preferably the chlorin e4 sodium according to the first aspect of the present invention and the pharmaceutical composition according to the fourth aspect of the present invention are suitable for the treatment of a disease characterised

by benign or malignant cellular hyperproliferation or by areas of neovascularisation; a benign or malignant tumour; early cancer; cervical dysplasia; soft tissue sarcoma; a germ cell tumour; retinoblastoma; Hodgkin's lymphoma; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system or bones.

Preferably the chlorin e4 sodium according to the first aspect of the present invention and the pharmaceutical composition according to the fourth aspect of the present invention are suitable for the treatment of a disease characterised by benign or malignant cellular hyperproliferation or by areas of neovascularisation.

Preferably the chlorin e4 sodium according to the first aspect of the present invention and the pharmaceutical composition according to the fourth aspect of the present invention are suitable for the treatment of a benign or malignant tumour.

Preferably the chlorin e4 sodium according to the first aspect of the present invention and the pharmaceutical composition according to the fourth aspect of the present invention are suitable for the treatment of early cancer; cervical dysplasia; soft tissue sarcoma; a germ cell tumour; retinoblastoma; lymphoma; Hodgkin's lymphoma; head and neck cancer; oral or mouth cancer; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system, bones, skin or pancreas. Preferably they are suitable for the treatment of early cancer; cervical dysplasia; soft tissue sarcoma; a germ cell tumour; retinoblastoma; Hodgkin's lymphoma; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system or bones. Preferably they are suitable for the treatment of lymphoma; Hodgkin's lymphoma; oral or mouth cancer; or cancer of the prostate, cervix, vaginal or other female adnexa, breast, naso-pharynx, lung, esophagus, kidney, liver, brain, lymphatic system, bones, skin or pancreas. Preferably they are suitable for the treatment of lymphoma; oral or mouth cancer; or cancer of the prostate, cervix, vaginal or other female adnexa, breast, naso-pharynx, lung, esophagus, bones or skin.

Preferably the chlorin e4 sodium according to the first aspect of the present invention and the pharmaceutical composition according to the fourth aspect of the present invention are suitable for use in photodynamic diagnosis.

Preferably the chlorin e4 sodium according to the first aspect of the present invention and the pharmaceutical composition according to the fourth aspect of the present invention are suitable for the detection of a disease characterised by benign or malignant cellular hyperproliferation or by areas of neovascularisation; a benign or malignant tumour; early cancer; cervical dysplasia; soft tissue sarcoma; a germ cell tumour; retinoblastoma; Hodgkin's lymphoma; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system or bones.

Preferably the chlorin e4 sodium according to the first aspect of the present invention and the pharmaceutical composition according to the fourth aspect of the present invention are suitable for the detection of an area that is affected by benign or malignant cellular hyperproliferation or by neovascularisation.

Preferably the chlorin e4 sodium according to the first aspect of the present invention and the pharmaceutical composition according to the fourth aspect of the present invention are suitable for the detection of a benign or malignant tumour.

Preferably the chlorin e4 sodium according to the first aspect of the present invention and the pharmaceutical composition according to the fourth aspect of the present invention are suitable for the detection of early cancer; cervical dysplasia; soft tissue sarcoma; a germ cell tumour; retinoblastoma; lymphoma; Hodgkin's lymphoma; head and neck cancer; oral or mouth cancer; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system, bones, skin or pancreas. Preferably they are suitable for the detection of early cancer; cervical dysplasia; soft tissue sarcoma; a germ cell tumour; retinoblastoma; Hodgkin's lymphoma; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system or bones. Preferably they are suitable for the detection of lymphoma; Hodgkin's lymphoma; oral or mouth cancer; or cancer of the prostate, cervix, vaginal or other female adnexa, breast, naso-pharynx, lung, esophagus, kidney, liver, brain, lymphatic system, bones, skin or pancreas. Preferably they are suitable for the detection of lymphoma; oral or mouth cancer; or cancer of the prostate, cervix, vaginal or other female adnexa, breast, naso-pharynx, lung, esophagus, bones or skin.

Preferably the chlorin e4 sodium according to the first aspect of the present invention and the pharmaceutical composition according to the fourth aspect of the present invention are suitable for the fluorescent or phosphorescent detection of the diseases listed above, preferably for the fluorescent or phosphorescent detection and quantification of the said diseases.

Preferably the chlorin e4 sodium according to the first aspect of the present invention and the pharmaceutical composition according to the fourth aspect of the present invention are adapted for administration simultaneous with or prior to administration of irradiation or sound, preferably for administration prior to administration of irradiation.

If the chlorin e4 sodium according to the first aspect of the present invention or the pharmaceutical composition according to the fourth aspect of the present invention are for use in photodynamic therapy or cytoluminescent therapy, then they are preferably adapted for administration 5 to 100 hours before the irradiation, preferably 6 to 72 hours before the irradiation, preferably 24 to 48 hours before the irradiation.

If the chlorin e4 sodium according to the first aspect of the present invention or the pharmaceutical composition according to the fourth aspect of the present invention are for use in photodynamic diagnosis, then they are preferably adapted for administration 3 to 60 hours before the irradiation, preferably 8 to 40 hours before the irradiation.

Preferably the irradiation used in the photodynamic therapy, cytoluminescent therapy or photodynamic diagnosis is electromagnetic radiation with a wavelength in the range of from 500nm to 1000nm, preferably from 600nm to 790nm. The electromagnetic radiation may be administered for about 5-60 minutes, preferably for about 15-20 minutes, at about 0.1-5W, preferably at about 1W. In one embodiment of the present invention, two sources of electromagnetic radiation are used (for example a laser light and an LED light), one source with a wavelength in the range of from 600nm to 690nm, preferably about 660nm, the other source with a wavelength in the range of from 700nm to 790nm, preferably about 760nm. In another embodiment of the present invention, two sources of electromagnetic radiation are used (for example a laser light and an LED light), both sources adapted to provide irradiation with a wavelength in the range of from 600nm to 690nm, preferably about 660nm, and irradiation with a wavelength in the range of from 700nm to 790nm, preferably about 760nm. In another embodiment of the present invention, the irradiation may be provided by a prostate, anal, vaginal, mouth and nasal device for insertion into a body cavity. In another embodiment of the present invention, the irradiation may be provided by interstitial light activation, for example, using a fine needle to insert an optical fibre laser into the lung, liver, lymph nodes or breast. In another embodiment of the present invention, the irradiation may be provided by endoscopic light activation, for example, for delivering light to the lung, stomach, colon, bladder or neck.

The pharmaceutical composition according to the fourth aspect of the present invention may be in a form suitable for oral, parenteral (including intravenous, subcutaneous, intramuscular, intradermal, intratracheal, intraperitoneal, intraarticular, intraabdominal, intracranial and epidural), transdermal, airway (aerosol), rectal, vaginal or topical (including buccal, mucosal and sublingual) administration. The pharmaceutical composition may also be in a form suitable for administration by enema or for administration by injection into a tumour. Preferably the pharmaceutical composition is in a form suitable for oral, parenteral or airway administration, preferably in a form suitable for oral or parenteral administration, preferably in a form suitable for oral administration.

In one preferred embodiment, the pharmaceutical composition is in a form suitable for oral administration. Preferably the pharmaceutical composition is provided in the form of a tablet, capsule, hard or soft gelatine capsule, caplet, troche or lozenge, as a powder or granules, or as an aqueous solution, suspension or dispersion. More preferably the pharmaceutical composition is provided in the form of an aqueous solution, suspension or dispersion for oral administration, or alternatively in the form of a freeze-dried powder which can be mixed with water before administration to provide an aqueous solution, suspension or dispersion for oral administration. Preferably the pharmaceutical composition is in a form suitable for providing 0.01 to 10 mg/kg/day of the chlorin e4 sodium, preferably 0.1 to 5 mg/kg/day, preferably about 3 mg/kg/day.

In another preferred embodiment, the pharmaceutical composition is in a form suitable for parenteral administration. Preferably the pharmaceutical composition is in a form suitable for intravenous administration. Preferably the pharmaceutical composition is provided in the form of an aqueous solution for parenteral administration, or alternatively in the form of a freeze-dried powder which can be mixed with water before administration to provide an aqueous solution for parenteral administration. Preferably the pharmaceutical composition is an aqueous solution or suspension having a pH of from 6 to 8.5.

In another preferred embodiment, the pharmaceutical composition is in a form suitable for airway administration. Preferably the pharmaceutical composition is provided in the form of an aqueous solution, suspension or dispersion for airway administration, or alternatively in the form of a freeze-dried powder which can be mixed with water before administration to provide an aqueous solution, suspension or dispersion for airway administration. Preferably the pharmaceutical composition is in a form suitable for providing 0.01 to 10 mg/kg/day of the chlorin e4 sodium, preferably 0.1 to 5 mg/kg/day, preferably about 3 mg/kg/day.

A fifth aspect of the present invention provides chlorin e4 sodium according to the first aspect of the present invention for use in the treatment of a disease characterised by benign or malignant cellular hyperproliferation or by areas of neovascularisation; a benign or malignant tumour; early cancer; cervical dysplasia; soft tissue sarcoma; a germ cell tumour; retinoblastoma; Hodgkin's lymphoma; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system or bones.

The fifth aspect of the present invention also provides chlorin e4 sodium according to the first aspect of the present invention for use in photodynamic therapy or cytoluminescent therapy. Preferably the phototherapeutic agent is suitable for the treatment of a disease characterised by benign or malignant cellular hyperproliferation or by areas of neovascularisation; a benign or malignant tumour; early cancer; cervical dysplasia; soft tissue sarcoma; a germ cell tumour; retinoblastoma; Hodgkin's lymphoma; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system or bones.

Preferably the chlorin e4 sodium or the phototherapeutic agent of the fifth aspect of the present invention is suitable for the treatment of a disease characterised by benign or malignant cellular hyperproliferation or by areas of neovascularisation.

Preferably the chlorin e4 sodium or the phototherapeutic agent of the fifth aspect of the present invention is suitable for the treatment of a benign or malignant tumour.

Preferably the chlorin e4 sodium or the phototherapeutic agent of the fifth aspect of the present invention is suitable for the treatment of early cancer; cervical dysplasia; soft tissue sarcoma; a germ cell tumour; retinoblastoma; lymphoma; Hodgkin's lymphoma; head and neck cancer; oral or mouth cancer; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system, bones, skin or pancreas. Preferably it is suitable for the treatment of early cancer; cervical dysplasia; soft tissue sarcoma; a germ cell tumour; retinoblastoma; Hodgkin's lymphoma; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system or bones. Preferably it is suitable for the treatment of lymphoma; Hodgkin's lymphoma; oral or mouth cancer; or cancer of the prostate, cervix, vaginal or other female adnexa, breast, naso-pharynx, lung, esophagus, kidney, liver, brain, lymphatic system, bones, skin or pancreas. Preferably it is suitable for the treatment of lymphoma; oral or mouth cancer; or cancer of the prostate, cervix, vaginal or other female adnexa, breast, naso-pharynx, lung, esophagus, bones or skin.

The fifth aspect of the present invention also provides chlorin e4 sodium according to the first aspect of the present invention for use in photodynamic diagnosis.

Preferably the photodiagnostic agent of the fifth aspect of the present invention is suitable for the detection of a disease characterised by benign or malignant cellular hyperproliferation or by areas of neovascularisation; a benign or malignant tumour; early cancer; cervical dysplasia; soft tissue sarcoma; a germ cell tumour; retinoblastoma; Hodgkin's lymphoma; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system or bones.

Preferably the photodiagnostic agent of the fifth aspect of the present invention is suitable for the detection of an area that is affected by benign or malignant cellular hyperproliferation or by neovascularisation.

Preferably the photodiagnostic agent of the fifth aspect of the present invention is suitable for the detection of a benign or malignant tumour.

Preferably the photodiagnostic agent of the fifth aspect of the present invention is suitable for the detection of early cancer; cervical dysplasia; soft tissue sarcoma; a germ cell tumour; retinoblastoma; lymphoma; Hodgkin's lymphoma; head and neck cancer; oral or mouth cancer; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system, bones, skin or pancreas. Preferably it is suitable for the detection of early cancer; cervical dysplasia; soft tissue sarcoma; a germ cell tumour; retinoblastoma; Hodgkin's lymphoma; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system or bones. Preferably it is suitable for the detection of lymphoma; Hodgkin's lymphoma; oral or mouth cancer; or cancer of the prostate, cervix, vaginal or other female adnexa, breast, naso-pharynx, lung, esophagus, kidney, liver, brain, lymphatic system, bones, skin or pancreas. Preferably it is suitable for the detection of lymphoma; oral or mouth cancer; or cancer of the prostate, cervix, vaginal or other female adnexa, breast, naso-pharynx, lung, esophagus, bones or skin.

Preferably the photodiagnostic agent of the fifth aspect of the present invention is suitable for the fluorescent or phosphorescent detection of the said diseases, preferably the fluorescent or phosphorescent detection and quantification of the said diseases.

Preferably the chlorin e4 sodium, the phototherapeutic agent or the photodiagnostic agent is adapted for administration simultaneous with or prior to administration of irradiation or sound, preferably for administration prior to

administration of irradiation.

If the chlorin e4 sodium or the phototherapeutic agent is for use in photodynamic therapy or cytoluminescent therapy, then it is preferably adapted for administration 5 to 100 hours before the irradiation, preferably 6 to 72 hours before the irradiation, preferably 24 to 48 hours before the irradiation.

If the photodiagnostic agent is for use in photodynamic diagnosis, then it is preferably adapted for administration 3 to 60 hours before the irradiation, preferably 8 to 40 hours before the irradiation.

Preferably the irradiation used in the photodynamic therapy, cytoluminescent therapy or photodynamic diagnosis is electromagnetic radiation with a wavelength in the range of from 500nm to 1000nm, preferably from 600nm to 790nm. The electromagnetic radiation may be administered for about 5-60 minutes, preferably for about 15-20 minutes, at about 0.1-5W, preferably at about 1W. In one embodiment of the present invention, two sources of electromagnetic radiation are used (for example a laser light and an LED light), one source with a wavelength in the range of from 600nm to 690nm, preferably about 660nm, the other source with a wavelength in the range of from 700nm to 790nm, preferably about 760nm. In another embodiment of the present invention, two sources of electromagnetic radiation are used (for example a laser light and an LED light), both sources adapted to provide irradiation with a wavelength in the range of from 600nm to 690nm, preferably about 660nm, and irradiation with a wavelength in the range of from 700nm to 790nm, preferably about 760nm. In another embodiment of the present invention, the irradiation may be provided by a prostate, anal, vaginal, mouth and nasal device for insertion into a body cavity. In another embodiment of the present invention, the irradiation may be provided by interstitial light activation, for example, using a fine needle to insert an optical fibre laser into the lung, liver, lymph nodes or breast. In another embodiment of the present invention, the irradiation may be provided by endoscopic light activation, for example, for delivering light to the lung, stomach, colon, bladder or neck.

**Brief description of the drawings**

Figure 1 shows a modified Jablonski diagram for a typical photosensitizer, outlining the principle photophysical processes of interest.

Figure 2 depicts schematically the metalation and demetalation of a porphyrin ring.

Figure 3 depicts schematically the reaction of 1,3-diphenylisobenzofuran (DPIBF) with two singlet oxygens ($^1O_2$).

Figure 4 shows the absorption spectrum of a chlorin e4 sodium solution (about 10 $\mu$M) in ethanol.

Figure 5 shows the absorption spectrum of a chlorin e4 sodium solution (about 10 $\mu$M) in water.

Figure 6 shows the absorption spectrum of a chlorin e4 sodium solution (about 10 $\mu$M) in water with 0.2 M detergent sodium dodecyl sulphate (SDS).

Figure 7 shows the fluorescence spectrum of a chlorin e4 sodium solution (about 10 $\mu$M) in ethanol. Excitation wavelength was 403 nm; the absorbance at this wavelength was 0.10; cuvette size 1cm.

Figure 8 shows the fluorescence spectrum of a chlorin e4 sodium solution (about 10 $\mu$M) in water. Excitation wavelength was 403 nm; the absorbance at this wavelength was 0.13; cuvette size 1 cm.

Figure 9 shows the fluorescence spectrum of a chlorin e4 sodium solution (about 10 $\mu$M) in water with 0.2 M detergent sodium dodecyl sulphate (SDS). Excitation wavelength was 403 nm; the absorbance at this wavelength was 0.13; cuvette size 1 cm.

Figure 10 shows the fluorescence excitation spectrum of a chlorin e4 sodium solution (about 10 $\mu$M) in ethanol. Fluorescence was measured at 710 nm; the absorbance at 403 nm was 0.15 in 1 cm; the optical path was 0.5 cm.

Figure 11 shows the absorption spectra of a solution of DPIBF and chlorin e4 sodium in ethanol before and 14 minutes after irradiation by red light (660 nm).

Figure 12 shows kinetic traces (1) and the spectrum (2) of singlet oxygen phosphorescence after pulsed laser excitation of chlorin e4 sodium in ethanol.

Figure 13 shows kinetic traces (1) and the spectrum (2) of singlet oxygen phosphorescence after pulsed laser excitation of chlorin e4 sodium in water.

Figure 14 shows kinetic traces of singlet oxygen phosphorescence after pulsed laser excitation of chlorin e4 sodium in water with 0.2 M detergent sodium dodecyl sulphate (SDS).

**Detailed description of the invention**

Metal insertion into the central part of a porphyrin or chlorin ring is known as metalation and the general chemical process is depicted in Figure 2. Porphyrins and chlorins can form complexes with a wide variety of metals (M). These metal complexes not only protect the inner nitrogen atoms from electrophilic reagents and strong bases, but also have a pronounced effect on the reactivity of the macrocycles. A common geometry of these complexes is octahedral, with the metal ions occupying the centre of the $N_4$ porphyrin plane and the metal ligands being in *trans* positions (Figure 2).

Chlorin e4 has nine side chains, two of which carry a carboxylic acid moiety. These carboxylic acid moieties can form salts with a wide variety of metals (M).

**Experimental details**

**Example - general synthetic details**

Method 1: 0.15-1mmol of chlorin e4 is dissolved in 60-100ml of methanol. To this solution, 15-20ml of sodium hydroxide (3M water solution) is added. The mixture is stirred until the synthesis is complete after about 6 hours. Then the mixture is filtered through a 45μm Millipore filter. The product is purified by flash chromatography with a methanol mobile phase. Then the solution is distilled and lyophilized. In a preferred embodiment of the present invention, the reaction is carried out at a temperature in the range of 25-60°C.

Method 2: 0.15-1mmol of chlorin e4 is dissolved in 60-100ml of methanol. To this solution, 15-20ml of sodium hydroxide (3M water solution) is added. Separately, 5-12mmol of thiamine pyrophosphate is dissolved in 40-60ml of water, and the latter solution is added to the previous one. The mixture is stirred until the synthesis is complete after about 6 hours. Then the mixture is filtered through a 45μm Millipore filter. The product is purified by flash chromatography with a methanol mobile phase. Then the solution is distilled and lyophilized. In a preferred embodiment of the present invention, the reaction is carried out at a temperature in the range of 25-60°C.

**Example - synthesis of chlorin e4 sodium**

Method 1: 0.5mmol (276mg) of chlorin e4 was dissolved in 60ml of methanol, and the solution was stirred slowly for 10 minutes at 40°C. 15ml of sodium hydroxide (3M water solution) was added, and stirring was continued for a further 6 hours at 60°C. Then the reaction mixture was filtered through a 45μm Millipore filter. The product was purified by flash chromatography on a dry silica gel column using a methanol mobile phase. Then the solution was reduced in volume on a rotary evaporator and the product was isolated as a dry powder by lyophilization in 85% yield.

The chlorin e4 sodium product was characterised by $^1H$ and $^{13}C$ NMR.

$^1H$ NMR ($D_2O$) (ppm): 8.82 (1H), 8.64 (1H), 6.52 (1H), 6.35 (1H), 5.25 (1H), 5.12 (1H), 4.68 (2H), 4.09 (3H), 3.52 (3H), 2.87 (1H), 2.71 (2H), 2.59 (3H), 2.44 (2H), 2.06 (1H), 1.82 (3H), 0.99 (6H).

$^{13}C$ NMR ($D_2O$) (ppm): 185.7 ($CO_2H$), 180.8 ($CO_2H$), 171.6 ($C_{qua}$=), 170.7 ($C_{quat}$=), 148.0 ($C_{quat}$=), 144.7 ($C_{quat}$=), 139.0 ($C_{quat}$=), 137.5 ($C_{quat}$=), 136.5 ($C_{quat}$=), 136.2 ($C_{quat}$=), 135.3 ($C_{quat}$=), 133.9 ($C_{quat}$=), 133.6 ($C_{quat}$=), 132.5 ($C_{quat}$=), 131.2 ($C_{quat}$=), 129.5 (CH=), 121.6 ($CH_2$=), 121.4 ($C_{quat}$=), 109.4 ($C_{quat}$=), 101.2 (CH=), 97.4 (CH=), 96.2 (CH=), 56.8 (CH), 50.9 (CH), 38.0 ($CH_2$), 34.5 ($CH_2$), 26.1 (Me), 21.7 (Me), 20.1 ($CH_2$), 18.7 (Me), 14.0 (Me), 12.6 (Me), 10.7 (Me).

Method 2: 0.5mmol (276mg) of chlorin e4 was dissolved in 60ml of methanol, and the solution was stirred slowly for 10 minutes at 40°C. 15ml of sodium hydroxide (3M water solution) was added, and stirring was continued for a further 20 minutes. Separately, 6mmol (378mg) of thiamine pyrophosphate was dissolved in 50ml of water, and the solution was stirred at 60°C. The thiamine pyrophosphate solution was added to the chlorin e4 solution, and the reaction mixture was stirred slowly for 6 hours at 60°C. Then the reaction mixture was filtered through a 45μm Millipore filter. The product was purified by flash chromatography on a dry silica gel column using a methanol mobile phase. Then the solution was reduced in volume on a rotary evaporator and the product was isolated as a dry powder by lyophilization in 85% yield.

The chlorin e4 sodium product was characterised by $^1H$ and $^{13}C$ NMR. The same results as for method 1 were obtained.

**Example** - ***in vitro* studies**

The following experiments were carried out to study the absorption and fluorescence spectra of chlorin e4 sodium, the quantum yields of chlorin e4 sodium fluorescence and the photosensitizing ability of chlorin e4 sodium in three systems - solutions in ethanol, pure water and water containing detergent sodium dodecyl sulfate (SDS).

*Absorption and fluorescence measurements*

Absorption spectra were recorded using computerized spectrophotometers Hitachi 3400 (Japan) and SF-2000 (Russia). Fluorescence spectra were measured with a computerized spectrofluorimeter Perkin-Elmer MPF-44B. 10 mm wide quartz cuvettes were used for absorption and fluorescence measurements. Fluorescence was detected at a right angle to the excitation beam. The spectral bandwidth of the monochromator was 3 nm. To avoid fluorescence re-absorption by the pigment molecules, diluted chlorin e4 sodium solutions were used with absorbance at 403 nm which did not exceed 0.2 cm in the 1 cm cuvette. The fluorescence quantum yields ($\Phi_F$) were determined using as a reference, solutions of TPPS (m-tetrakis(p-sulfonatophenyl)porphine) in ethanol. According to the literature, the most likely fluorescence quantum yield of TPPS in air-saturated water and ethanol is equal to 0.08 (K. Kalyanasundaram and M. Neumann-Spallart, Photophysical and redox properties of water soluble porphyrins in aqueous media, J. Phys. Chem., 1982, 86, 5163-5169; and A.T. Gradyushko, A.N. Sevchenko, K.N. Solovyov and M.P. Tsvirko, Energetics of photophysical processes in chlorophyll-like molecules, Photochem. Photobiol., 1970, 11, 387-400). The fluorescence of the samples was excited by the monochromatic light corresponding to the maximum of the Soret bands: 415 nm for TPPS in ethanol and 413 nm for TPPS in water, and 403 nm for chlorin e4 sodium in water and ethanol. The monochromator bandwidth was 3 nm. The fluorescence quantum yields ($\Phi_F$) were calculated using the following equation:

$$\Phi_F = \Phi_{TPP} \cdot \frac{S_c \cdot (1-10^{-D_{TPP}})/(1-10^{-D_c})}{S_{TPP}}$$

where $\Phi_{TPP}$ is the quantum yield of TPPS fluorescence, $\mathbf{S_c}$ is the square under the fluorescence spectrum of the chlorin e4 sodium sample, $\mathbf{S_{TPP}}$ is the square under the fluorescence spectrum of TPPS, and $\mathbf{D_c}$ and $\mathbf{D_{TPP}}$ are absorbance (optical density) of the chlorin e4 sodium and TPPS respectively at the excitation wavelengths. The obtained results were collated using relative units of intensity of the excited light at the excitation wavelengths. The intensities of the monochromatic light were measured using a calibrated silicon detector from Optical Power Meter System ThorLabs (Karlsfeld, Germany).

*Detection of singlet oxygen phosphorescence*

Singlet oxygen phosphorescence was detected using measurement of infrared phosphorescence at 1270 nm corresponding to the transition of oxygen molecules from the singlet to the triplet state. Phosphorescence was recorded at room temperature with the laser phosphorescence spectrometer described at A.A. Krasnovsky Jr., Photodynamic action and singlet oxygen, Biofizika, 2004, 49, 305-321; and A.A. Krasnovsky Jr., Singlet oxygen and primary mechanisms of photodynamic therapy and photodynamic diseases, In: Photodynamic therapy at the cellular level, ed. A.B. Uzdensky, Research Singpost, Kerala, India, 2007, 17-62. For excitation, nanosecond pulses of 511 nm radiation from a copper-vapour laser (Troitzk, Physics Institute of the Russian Academy of Science) were employed. Laser radiation passed additionally through a green light filter SZS-22 (4 mm), which transmitted laser light and cut off IR laser radiation caused by heating of the laser tube. Pulse duration was 20 ns, pulse energy varied from 18 to 40 μJ and the pulse repetition rate was 12 kHz. The laser light was collimated on the surface of a 1 cm rectangular quartz cuvette. The diameter of the illuminated spot on the surface of the cell was 5 mm. For kinetic measurements another spectrometer was used without a monochromator. In this case, phosphorescence resulting from the right angle to the excitation beam was focused on the photocathode of a cooled S-1 photomultiplier (FEU-112) through a cut-off IR light filter and an interference filter with the transmission maximum at 1270 nm. The phosphorescence spectra were measured using a MS-80 high throughput grid monochromator. In this case, phosphorescence was focused on the entry slit of this monochromator. The bandwidth of the monochromator corresponded to a 20 nm spectral interval. The monochromatic light was focused on the photocathode of a cooled S-1 photomultiplier (FEU-83). The photomultiplier signal was registered using time-correlated single photon counting and averaged over many laser pulses. In most experiments, one-channel on time was equal to 81 ns. When the pigment bleaching reached 10%, the solution was replaced by a fresh one and measurement was continued. As chlorin e4 sodium bleached rather rapidly, the solutions were changed after each 60-180 s irradiation. Reasonable phosphorescent curves were obtained after 10-20 minutes signal accumulation and therefore samples were

changed many times. As described previously (D.A. Butorina, A.A. Krasnovsky Jr. and A.V. Priezzev, Investigation of the kinetic parameters of singlet molecular oxygen in aqueous porphyrin solutions, Influence of detergents and the quencher sodium azide, Biofizika, 2003, 48, 189-196; A.A. Krasnovsky Jr., Luminescence and photochemical studies of singlet oxygen photonics, J. Photochem. Photobiol. A: Chem., 2008, 196, 210-218; and N. Kuznetsova, D. Makarov, O. Yuzhakova, A. Strizhakov, Y. Roumbal, L. Ulanova, A. Krasnovsky and O. Kaliya, Photophysical properties and photodynamic activity of octacationic oxotitanium(IV) phthalocyanines, Photochemical Photobiological Sciences, 2009, 8, 1724-1733), for computer analysis the following two-exponential equation was used:

$$I(t) = I_0 \cdot [\exp(-k_{decay} \cdot t) - \exp(-k_{rise} \cdot t)] + n$$

where $\mathbf{k_{decay}}$ and $\mathbf{k_{rise}}$ are the rate constants of the decay and rise phases of the phosphorescence curves after a laser pulse, **n** is the off noise of the photomultiplier, $\mathbf{I_0}$ is the zero-time phosphorescence intensity, and **I(t)** is the phosphorescence intensity at time **t.** To reduce contribution of the luminescence of the pigment in the quartz cuvette, which was observed shortly after laser pulses, readings were taken starting from 400 ns after laser pulses in ethanol and 1 μs in water. In addition, phosphorescence and kinetic traces were measured also from the control chlorin e4 sodium solutions containing high concentration of sodium azide, which completely quenched singlet oxygen. This procedure revealed a strong rapidly decaying component of delayed luminescence in the chlorin e4 sodium solutions, which was not so easily quenched by sodium azide. The luminescent curves obtained from the control solutions were approximated by exponentials and subtracted from the experimental kinetic traces obtained for the chlorin e4 sodium solutions where sodium azide was not added.

For measurements, Origin 6.0 software was used. This software operates using the least-square approximation method. Origin allowed variation of the parameters $I_0$, $k_{decay}$, $k_{rise}$ and n in order to obtain their values corresponding to the minimum coefficient $x^2$. In chlorin e4 sodium solutions, measurements always yielded $k_{decay}$, which was somewhat shorter than in the reference solutions of TPPS in the same solvents. In the solvents used in this study, the lifetime of singlet oxygen, which is responsible for $k_{decay}$, is independent of the pigments. Therefore this result indicates that "the azide control" does not allow to fully eliminate the rapidly decaying component from the overall decay curves. It is possible that luminescence responsible for this component is partially quenched by sodium azide. In order to eliminate the error caused by this effect, for final measurements of phosphorescent curves in chlorin e4 sodium solutions, fixed $k_{decay}$ obtained in the TPPS solutions was used. In this case as Origin indicated, the correlation coefficient $R^2$ was not less than 98% and error of $k_{rise}$ determination was less than ± 5% in all solvents. The quantum yields ($\Phi_\Delta$) of singlet oxygen photogeneration by chlorin e4 sodium were determined from comparison of the overall singlet oxygen phosphorescence intensities in the chlorin e4 sodium solutions and the reference solutions of TPPS in the same solvents. According to the literature, the most probable values of quantum yields of singlet oxygen photogeneration by TPPS are 0.6 in water and 0.7 in ethanol and detergent containing water (F. Wilkinson, W.P. Helman and A.B. Ross, Quantum yields of the photosensitized formation of the lowest electronically excited singlet states of molecular oxygen, J. Phys. Chem. Ref. Data, 1993, 22(2), 113-262; and C. Tanielian, C. Wolf and M. Esch, Singlet oxygen production in water: Aggregation and charge transfer effects, J. Phys. Chem., 1996, 100, 6555-6560). The overall phosphorescence intensity was calculated by integrating the phosphorescence kinetic traces. The absorbance of the pigment solutions at 511 nm was usually about 0.1-0.2 in 5 mm cuvette. This corresponded to a pigment concentration equal to about 20 μM.

*Detection of singlet oxygen using chemical conjugates*

The second method of singlet oxygen detection was based on photosensitized oxygenation of the singlet oxygen conjugate 1,3-diphenylisobenzofuran (DPIBF) (Acros Organics, United Kingdom). DPIBF chemically binds to two singlet oxygens ($^1O_2$) (Figure 3). This method was applied to chlorin e4 sodium solutions in ethanol. The major absorption band of DPIBF is located at 414-415 nm. The molar absorption coefficient in this maximum, $\varepsilon_{DPIBF}$, is known to be 23500 $M^{-1}cm^{-1}$. Tested solutions were irradiated by the monochromatic light from a xenon lamp using the excitation system of the spectrofluorimeter Perkin-Elmer MPF-44B, the spectral width of the monochromator bandwidth corresponding to 5 nm. Radiant power was measured using the calibrated detector ThorLabs PM-100 (Germany). The singlet oxygen quantum yields ($\Phi_\Delta$) were calculated according to the equation:

$$\Phi_\Delta = \frac{\eta_P \cdot \dfrac{\Delta D_{DPIBF}}{\varepsilon_{DPIBF} \cdot l \cdot t_{irr}} \cdot V \cdot N_A}{\dfrac{I_{irr}}{h\nu(1-T)}}$$

where $\Delta D_{DPIBF}$ is the reduction in the optical density of DPIBF at 414 nm during the irradiation time, $\varepsilon_{DPIBF}$ is the molar absorption coefficient of DPIBF at 414 nm, **1** is the optical path equal to 1 cm, **V** is the volume of tested solutions (usually 1.2-1.5 ml), $N_A$ is the Avogadro constant, $I_{irr}$ is the radiant power (in W) of the monochromatic light from the excitation system of the Perkin-Elmer spectrofluorimeter that includes the xenon lamp and the excitation monochromator with the bandwidth corresponding to the 5 nm wavelength interval, **hv** is the energy of one photon in J, **(1-T)** is the absorption factor of the tested solutions, $t_{irr}$ is the irradiation time in seconds, and $\eta_P$ is the fraction of $^1O_2$ molecules which is captured by DPIBF in the studied solutions. This parameter was calculated using known kinetic equations for singlet oxygen consumption by chemical conjugates and the rate constant of singlet oxygen reaction with DPIBF equal to $10^9$ $M^{-1}s^{-1}$ (see A.A. Krasnovsky Jr., Y.V. Roumbal and A.A. Strizhakov, Rates of 1O2 production upon direct excitation of molecular oxygen by 1270 nm laser radiation in air-saturated alcohols and micellar aqueous dispersions, Chem. Phys. Lett., 2008, 458, 195-199; and references therein).

*Results*

Absorption spectra of chlorin e4 sodium in ethanol, pure water and water containing detergent sodium dodecyl sulfate (SDS) are shown in Figures 4-6. The major absorption bands are listed in Table 1. The strongest are the Soret band at 403 nm and the red band at 654-662 nm. Analysis of the absorption spectra suggests that chlorin e4 sodium was mostly monomeric in all solvents. In water having no detergent, the red absorption maximum was slightly shifted to the shorter wavelength region (from 662 to 654 nm) and a weak shoulder at about 665-670 nm was seen. In addition, the observed absorbance in the green region was increased compared to ethanol and detergent containing solutions. These data indicate that the solution in water having no detergent may contain aggregated (dimeric and oligomeric) molecules of chlorin e4 sodium, which disaggregate in the presence of the detergent.

Table 1 - Positions of the major absorption and fluorescence bands of chlorin e4 sodium solutions

| Solvents | Absorption maxima (nm ± 1 nm) | Fluorescence maxim (nm ± 1 nm) |
|---|---|---|
| Ethanol | 403, 504, 606, 662 | 666, 720 |
| Water | 402, 504, 602, 654 | 657, 710 |
| Water + 0.2 M SDS | 403, 504, 608, 662 | 665, 720 |

Fluorescence measurements supported this assumption (Figures 7-9). The fluorescence spectra were rather similar in ethanol and SDS containing water. Figure 10 shows the excitation spectrum of chlorin e4 sodium fluorescence in ethanol. It is seen that it practically coincides with the absorption spectrum of this pigment. This observation indicates that fluorescence belongs to chlorin e4 sodium. In water without SDS, the fluorescence maximum was shifted to shorter wavelengths and a shoulder at about 710 nm became markedly stronger than in ethanol. The fluorescence quantum yield was found to be highest in ethanol, slightly less in SDS containing water and the smallest in water without SDS. These data indicate that aggregation of chlorin e4 sodium occurs in water. However, the chlorin e4 sodium fluorescence quantum yield was still high in water without SDS, showing that an appreciable amount of monomeric chlorin e4 sodium molecules are present in aqueous solutions without detergent. These experiments show that chlorin e4 sodium is a highly fluorescent pigment and emits a large quantity of photons. Hence chlorin e4 sodium can be used efficiently as a fluorescent pigment for photodynamic diagnosis.

Singlet oxygen generation was studied in ethanol solutions using both chemical singlet oxygen conjugate DPIBF and measurement of chlorin e4 sodium photosensitized phosphorescence of singlet oxygen at 1270 nm. Figure 11 illustrates the experiment with DPIBF. The absorption maximum of DPIBF lies at 420 nm. It is seen from Figure 11 that irradiation of an ethanol solution containing both chlorin e4 sodium and DPIBF by red light (660 nm), which is absorbed by chlorin e4 sodium but not by DPIBF, caused bleaching of the DPIBF maximum at 420 nm. This effect results from reaction of DPIBF with singlet oxygen, which is formed upon irradiation of chlorin e4 sodium. The quantum yield of singlet oxygen generation obtained from this experiment was shown to be 0.68 ± 0.05 in ethanol. Kinetic traces of chlorin e4 sodium photosensitized phosphorescence of singlet oxygen after laser pulses are shown in Figures 12-14. Table 2 summarizes kinetic parameters of the phosphorescent curves. The rise time reflects the rate of singlet oxygen generation by triplet pigment molecules. It corresponds with the lifetime of the pigment triplet state in air-saturated pigment solution. In ethanol, the rise time was equal to 300 ns. The decay time corresponds with the singlet oxygen lifetime in ethanol (13.5 $\mu$s). The singlet oxygen quantum yield in ethanol determined using these kinetic curves was equal to 0.63 ± 0.05, which is close to that obtained using DPIBF. Thus both methods provided similar results. Table 2 indicates that chlorin e4 sodium is a potent photosensitizer of singlet oxygen formation in alcoholic as well as aqueous systems, though in water without SDS the photosensitizing activity was lower than in ethanol. This effect may have been due to partial

aggregation of pigment molecules, which is known to lead to the loss of photosensitizing activity. These experiments show that chlorin e4 sodium has strong photosensitizing ability in organic solvent (alcohol) and in aqueous media, with a high singlet oxygen quantum yield. Hence chlorin e4 sodium can be used efficiently in photodynamic therapy.

Table 2 - Kinetic parameters of photosensitized singlet oxygen phosphorescence in air saturated chlorin e4 sodium solutions

| Solvents | Rise time, μs | Decay time, μs |
|---|---|---|
| Ethanol | 0.3 ± 0.3 | 13.5 ± 0.3 |
| Water | 1.8 ± 0.4 | 3.3 ± 0.3 |
| Water + 0.2 M SDS | 1.2 ± 0.3 | 3.85 ± 0.3 |

*Conclusion*

Chlorin e4 sodium shows strong fluorescence and strong photosensitizing ability in organic solvent (alcohol) and in aqueous media. In water, the chlorin e4 sodium photosensitizing activity decreases due to aggregation of pigment molecules, but is still high. Addition of detergent causes disaggregation and an increase of both fluorescence and photosensitizing ability. This shows that chlorin e4 sodium is active in both hydrophilic and aqueous media. Therefore this pigment is promising for application in photodynamic medicine.

**Example** - ***in vivo*** **studies**

The following cancer patients were treated successfully with chlorin e4 sodium. Most of the patients were late stage metastatic cancer patients who had previously unsuccessfully used traditional treatment therapies. Most of the patients were in poor health prior to treatment with chlorin e4 sodium.

*Example 1*

A 70 year old female patient with invasive ductile breast cancer and lymph node metastases in the right arm pit, was treated successfully. Prior to treatment a large cancer mass in the right breast was observed and a small cancer mass in the right arm pit. The treatment consisted of three courses. For each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm.

Following treatment, doppler ultrasound analysis showed that the large cancer mass in the breast showed almost no vascularity, no blood vessels leading to it. The cancer mass had not reduced in size, however, the outline of the mass was solid and smooth, indicating an inactive, non-growing tumour. The smaller lymph node cancer mass also showed almost no vascularity and it had reduced in size from 1.9cm to 1cm.

Subsequently the patient was treated with two further courses. Again, for each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm. Additionally an interstitial light treatment using a fine needle was performed on the breast with an optical fibre laser having a wavelength of 660nm and 760nm.

Doppler ultrasound analysis obtained after the fourth treatment course, showed that the large cancer mass in the breast had reduced in size by about 25%. PET/CT scans obtained after the fifth and final treatment course, showed a complete response in the breast and lymph nodes. After each treatment course, an inflammatory response was observed in the breast tumour site, which is typical as the immune system attacks the tumour. Towards the end of the second treatment course, the patient experienced a burning sensation in the tumour site of her breast. The patient is currently alive and well, two years and three months after starting her treatment and five months after completion of her treatment.

*Example 2*

A 62 year old male patient with naso pharyngeal carcinoma with extension to a lymph node in the left neck, was treated successfully. The treatment consisted of three courses. For each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm.

Immediately following treatment, a biopsy of the treated lymph node of the neck showed a benign or non-cancerous lymph node, no cancer cells were detected. This was confirmed by another biopsy ten months after treatment. A comparison of CT scans, obtained one month before and ten months after treatment, showed no spread of tumours and that the size of the tumours was reduced.

Subsequently the patient was treated with a further course. Again, for the course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm.

Ultrasound images and biopsies obtained after the final treatment course, showed a complete response. The patient suffered no side effects, but an inflammatory response was observed after each treatment, which is typical as the immune system attacks the tumour. He is currently alive and well, two years and six months after starting his treatment and five months after completion of his treatment.

*Example 3*

A 71 year old male patient with advanced metastatic kidney disease with metastases in the gall bladder, liver and lung, was treated successfully. The treatment consisted of three courses. For each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm.

PET/CT scans, obtained just before treatment and two months, three months, five months and 15 months after treatment, showed that throughout the patient's body some tumours disappeared altogether and the remaining tumours reduced in size. No further metastasis was observed.

Subsequently the patient was treated with a further two courses. Again, for each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm. Additionally an interstitial light treatment using a fine needle was performed on the liver and lung with an optical fibre laser having a wavelength of 660nm and 760nm.

PET/CT scans again showed a good treatment response. The patient suffered no side effects from the treatment. Unfortunately the patient has recently died. Prior to the five treatment courses, he was an advanced stage cancer patient with a life expectancy of only six months, but he managed to live an active life for two years and four months.

*Example 4*

A 20 year old female patient with stage IVB Hodgkin's lymphoma with metastases in the liver, lymphatic system, spleen, lung and bones, was treated successfully. The treatment consisted of three courses. For each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm.

CT scans showed that in the areas of the patient's body that were treated with laser and LED light so far, some tumours disappeared altogether and some tumours reduced in size.

Subsequently the patient was treated with a further two courses. Again, for each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm.

PET/CT scans again showed a good treatment response. Unfortunately the patient has recently died. Prior to the five treatment courses, she was in an extremely poor condition, suffering from extreme weight loss and lethargy, too weak to walk, and requiring constant pain relief. Before her treatment, her life expectancy was only two weeks, but she managed to live for two years and two months, at least part of it active.

*Example 5*

A 82 year old male patient with lung cancer was treated successfully. The treatment consisted of one course of five doses of 62.5-187.5mg chlorin e4 sodium administered orally over the course of eight days. 24-48 hours after each oral dose of chlorin e4 sodium, the patient was treated with laser and LED light having a wavelength of 660nm and 760nm.

Three months after treatment, the patient was coughing up blood and tumour tissue. A CT scan showed tumour breakdown.

*Example 6*

A 46 year old male patient with lung cancer was treated successfully. The treatment consisted of three courses.

For each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm.

A CT scan, obtained immediately after the last treatment course, showed complete recovery from lung cancer.

*Example 7*

A 54 year old female patient with advanced pancreatic cancer with metastases in the liver, was treated successfully. Prior to treatment the patient was in poor health generally and suffering from extreme weight loss, pain and other typical symptoms of advanced pancreatic cancer. The treatment consisted of three courses. For each course, five doses of 62.5-187.5mg chlorin e4 sodium were administered orally over the course of eight days. About 24 hours after each oral dose of chlorin e4 sodium, the patient was treated with laser and LED light having a wavelength of 660nm and 760nm.

PET/CT scans of the pancreas, obtained before and after treatment, showed improvements and a good treatment response in the pancreatic area. The patient had no photosensitive reaction and suffered less pain. Unfortunately bowel blocking and malnutrition caused the patient's death.

*Example 8*

A 67 year old female patient with non-small cell lung cancer with metastases in the lymph nodes and bones, was treated successfully. The treatment consisted of two courses. For each course, five doses of 62.5-187.5mg chlorin e4 sodium were administered orally over the course of eight days. About 24 hours after each oral dose of chlorin e4 sodium, the patient was treated with laser and LED light having a wavelength of 660nm and 760nm.

PET/CT scans of the lungs, obtained before and after treatment, showed a remarkable response to treatment, with many tumours disappearing and the remaining tumours showing no or low metabolic activity after treatment. The patient suffered no side effects and her health improved. She is currently alive and well, 10 months after starting her treatment and seven months after completion of her treatment.

*Example 9*

A 54 year old female patient with breast cancer in the right breast was treated successfully. The treatment consisted of three courses. For each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm.

Mammography, ultrasound and PET/CT scans of the breasts, obtained before and after treatment, showed a complete response. The patient suffered no side effects. She is currently alive and well, one year and seven months after starting her treatment and 10 months after completion of her treatment.

*Example 10*

A 33 year old male patient with naso pharyngeal carcinoma with metastases in the lymph nodes of the left neck and in the lung, was treated successfully. The treatment consisted of five courses. For each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm. Additionally an interstitial light treatment using a fine needle was performed on the left lung to a tumour that was large in size with an optical fibre laser having a wavelength of 660nm and 760nm.

PET/CT scans showed a very good treatment response. The patient is currently alive and well, one year and five months after starting his treatment and two months after completion of his treatment.

*Example 11*

A 44 year old female patient with lung cancer with metastases in the lymph nodes and sacrum bone, was treated successfully. The patient had an operation to remove the primary tumour of the lung and completed four cycles of chemotherapy. Subsequent PET/CT scans showed tumours in the lymph nodes of the neck, the lymph nodes in the left arm pit and the sacrum bone. The treatment consisted of five courses. For each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm. Additionally, for two of the treatment courses, an interstitial light treatment using a fine needle was performed on the sacrum bone with

an optical fibre laser having a wavelength of 660nm and 760nm. Additionally, mesothelium bone growth therapy was administered for the last two treatment courses.

PET/CT scans showed a complete response, with no active tumours in the patient's body. The patient is currently alive and well, 10 months after starting her treatment and four months after completion of her treatment.

*Example 12*

A 52 year old male patient with brain cancer with metastases in the lung and lymph nodes, was treated successfully. An MRI scan showed a large tumour mass in the brain. The patient had an operation to debulk the tumour pressing against his brain, but he did not have any chemotherapy or radiotherapy. Subsequent PET/CT scans showed active tumours in the brain with metastases in both lungs and the lymph nodes in the right cervical and right supraclavicular regions. The patient experienced occasional epileptic seizures and severe headaches. The treatment consisted of two courses. For each course, five doses of 62.5-187.5mg chlorin e4 sodium were administered orally over the course of eight days. 24-48 hours after each oral dose of chlorin e4 sodium, the patient was treated with laser and LED light having a wavelength of 660nm and 760nm.

PET/CT scans showed a complete response in the lungs and no metabolic activity in the area of the brain tumour. The lymph nodes were not yet clear, and the patient will undergo a third treatment course.

**Example - clinical study with patients suffering from advanced non-small cell lung cancer**

Through pathology or cytology diagnosis, 66 advanced non-small cell lung cancer patients were selected and randomly divided into two groups. Group A had 32 patients who were treated with chlorin e4 sodium. Group B had 34 patients who were treated with both chemotherapy and radiotherapy.

For group A, chlorin e4 sodium was administered at a dosage of 2 mg per kilogram of body weight, with two thirds administered by inhalation and one third administered orally. After the irradiation target(s) had been determined, an optical fibre for laser treatment purposes was guided into the lung tumor(s) via an interstitial procedure. Irradiation light intensity was 200 J/cm$^2$, irradiation power was 1,000 mW, and irradiation lasted for eight to ten minutes. Laser irradiation into the tumor was two to five centimeters in depth. Irradiation was either performed phase by phase (subparagraph treatment) based on tumor size or performed repeatedly.

For group B, for the radiotherapy, a 15 MV X-ray radiation therapy irradiation field covered primary lung tumor lesion(s) and mediastinal lymphatic drainage area, with a dosage of 65-70 Gy, administered on every first day in a week of radiotherapy. For the chemotherapy, 20 mg cisplatin was administered by intravenous application.

One and two years survival rates for groups A and B were 93.75%, 70.60% and 68.75, 32.35% respectively, the difference was statistically significant ($P<0.05$). The combined complete and partial remission rates for groups A and B were 56.20% and 21.30%, the rate of group A was significantly higher than that of group B, the difference was statistically significant ($P<0.05$).

**Example - study with mice with subcutaneous S180 sarcoma**

In this experiment the effects of photodynamic therapy mediated by three different photosensitizers, namely Duteroporphyrin, chlorin e4 sodium and 5-ALA, on mouse S180 sarcoma were observed. Duteroporphyrin and 5-ALA are porphyrin derivatives.

40 Kunming mice with well grown subcutaneous S180 sarcoma were randomly divided into four groups: (A) Duteroporphyrin group (10mg/kg), (B) chlorin e4 sodium group (20mg/kg), (C) 5-ALA group (100mg/kg) and (D) blank control group. The mice in groups A and B were subjected to irradiation 8-10 hours after tail vein injection of the photosensitizers, while the mice in group C were subjected to irradiation 3 hours after tail vein injection of the photosensitizer. A laser with a wavelength of 630nm was used for groups A and C, while a duel-frequency laser with wavelengths of 660nm and 760nm was used for group B. The local tumour sites were irradiated vertically, with the diameter of the laser spots being 1.2-1.5cm. The whole tumour and 3-5mm of normal tissue around the tumour were covered by the laser. The lasers were applied to groups A, B and C for 20 minutes, with a power density of 150mW/cm$^2$ and an energy density of 180J/cm$^2$. The mice in control group D were not subjected to irradiation.

After the treatments, tumour changes in appearance were observed. Black eschars were seen in groups A and B after treatments, while the eschars observed in group C were thinner and brownish red in colour. Skins and fur were fully recovered in all three treatment groups after the eschars had fallen off; group C was the fastest in skin repairing.

From the tumour growth curves it could be seen that there was no obvious difference in sizes of tumours between the treatment groups A, B and C and the control group D six days after the treatments. This was possibly caused by the oedema and eschars caused by the treatment which occurred at the irradiation sites. Observation of the tumours' sizes 21 days after the treatments indicated that the mean volume differences between all treatment groups

A, B and C and the control group D were statistically significant ($P<0.05$), the mean volume being statistically significantly smaller in treatment groups A, B and C than the control group D. The mean volume difference between group B and group C was also statistically significant ($P<0.05$), the mean volume being statistically significantly smaller in group B than group C.

Moreover, all treatment groups showed statistically significant tumour inhibition compared to the control group D, and group B showed a statistically significantly better therapeutic effect than group C ($P<0.05$). The tumour inhibition rates of groups A, B and C were respectively 62.5%, 75.4% and 37.2%.

Based on the analysis of apoptotic and necrotic tumour cells by FCM 24 hours after treatments, the proportions of apoptotic and necrotic tumour cells were a lot higher and living cells were obviously fewer in all treatment groups A, B and C than in the control group D, indicating that all treatment groups showed clear effects in killing cancer cells.

The pathological sections of the tumour tissues were observed via HE staining. The pathological changes of tumour tissues in the treatment groups A, B and C 24 hours after the treatment included: a wide range of tumour necrosis, tumour vascular disruption and neutrophil infiltration.

The mechanisms of killing tumour cells by photodynamic therapy include directly killing tumour cells, damaging tumour vessels and stimulating patients' immune systems. These factors may benefit each other and they are all long-term tumour-controlling mechanisms.

To sum up, this study verified that photodynamic therapy with Duteroporphyrin, chlorin e4 sodium and 5-ALA can inhibit the growth of S180 sarcoma in mice. Chlorin e4 sodium had a similar or slightly better therapeutic effect than Duteroporphyrin, and a statistically significantly better therapeutic effect than 5-ALA.

It will be understood that the present invention has been described above by way of example only.

## Claims

1. Chlorin e4 sodium, having the structure:

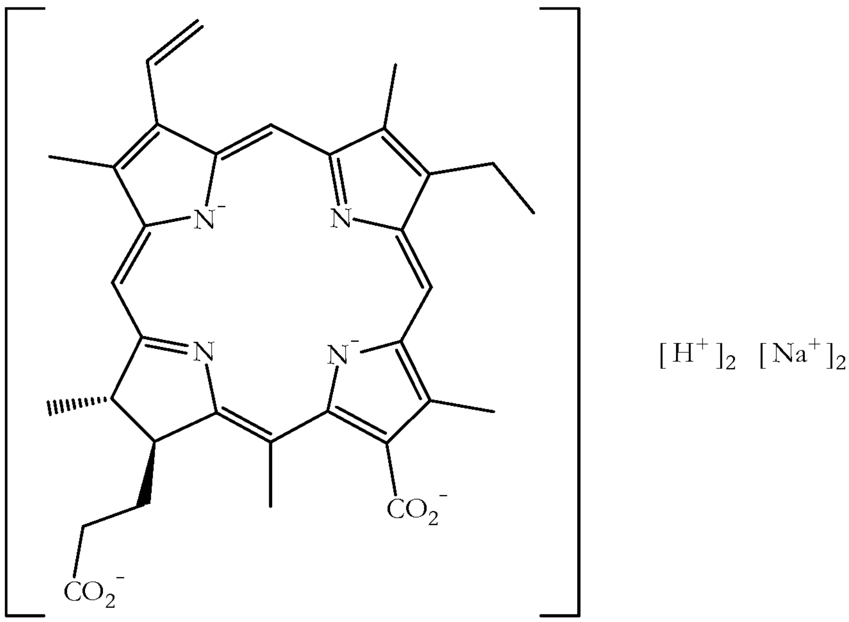

.

2. The chlorin e4 sodium of claim 1, having the structure:

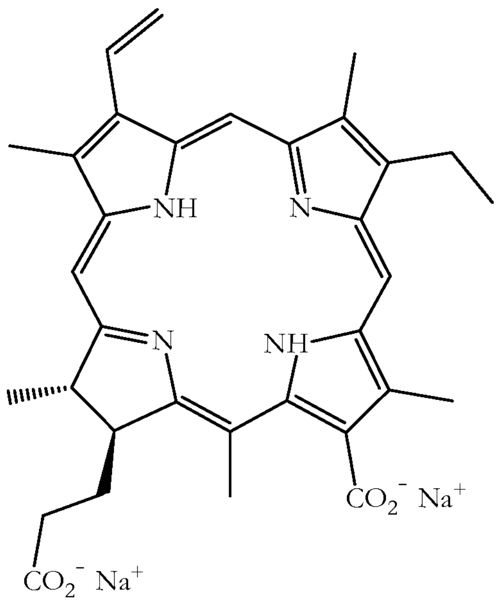

3. A process of preparing the chlorin e4 sodium of claim 1 or 2, comprising the step of reacting chlorin e4 with a sodium compound.

4. The process of claim 3, wherein the sodium compound is sodium hydroxide.

5. The process of claim 3 or 4, wherein chlorin e4 is reacted with the sodium compound in the presence of a base, preferably an amine base, preferably thiamine pyrophosphate.

6. The process of any one of claims 3 to 5, wherein chlorin e4, the sodium compound and the optional base are used in a molar ratio of 0.15-1 : 45-60 : 0-12.

7. The chlorin e4 sodium of claim 1 or 2, for use in medicine.

8. The chlorin e4 sodium of any one of claims 1, 2 or 7, for use in photodynamic therapy or cytoluminescent therapy.

9. The chlorin e4 sodium of any one of claims 1, 2, 7 or 8, for use in the treatment of:

(i) a disease **characterised by** benign or malignant cellular hyperproliferation or by areas of neovascularisation; and/or
(ii) a benign or malignant tumour; and/or
(iii) early cancer; cervical dysplasia; soft tissue sarcoma; a germ cell tumour; retinoblastoma; lymphoma; Hodgkin's lymphoma; head and neck cancer; oral or mouth cancer; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system, bones, skin or pancreas.

10. The chlorin e4 sodium of any one of claims 1, 2 or 7, for use in photodynamic diagnosis.

11. The chlorin e4 sodium for use of any one of claims 7 to 10, wherein the chlorin e4 sodium is adapted for administration prior to administration of irradiation, preferably wherein the irradiation is electromagnetic radiation with a wavelength in the range of from 500nm to 1000nm.

12. A pharmaceutical composition comprising the chlorin e4 sodium of any one of claims 1-2 or 7-11 and a pharmaceutically acceptable carrier or diluent.

13. The pharmaceutical composition of claim 12, for use in photodynamic therapy or cytoluminescent therapy.

**14.** The pharmaceutical composition of claim 12 or 13, for use in the treatment of:

(i) a disease **characterised by** benign or malignant cellular hyperproliferation or by areas of neovascularisation; and/or
(ii) a benign or malignant tumour; and/or
(iii) early cancer; cervical dysplasia; soft tissue sarcoma; a germ cell tumour; retinoblastoma; lymphoma; Hodgkin's lymphoma; head and neck cancer; oral or mouth cancer; or cancer of the blood, prostate, cervix, uterus, vaginal or other female adnexa, breast, naso-pharynx, trachea, larynx, bronchi, bronchioles, lung, hollow organs, esophagus, stomach, bile duct, intestine, colon, colorectum, rectum, bladder, ureter, kidney, liver, gall bladder, spleen, brain, lymphatic system, bones, skin or pancreas.

**15.** The pharmaceutical composition of claim 12, for use in photodynamic diagnosis.

**16.** The pharmaceutical composition of any one of claims 12 to 15, wherein the pharmaceutical composition is adapted for administration prior to administration of irradiation, preferably wherein the irradiation is electromagnetic radiation with a wavelength in the range of from 500nm to 1000nm.

**17.** The pharmaceutical composition of any one of claims 12 to 16, wherein the pharmaceutical composition is in a form suitable for oral, parenteral (including intravenous, subcutaneous, intramuscular, intradermal, intratracheal, intraperitoneal, intraarticular, intraabdominal, intracranial and epidural), transdermal, airway (aerosol), rectal, vaginal or topical (including buccal, mucosal and sublingual) administration, preferably wherein the pharmaceutical composition is in a form suitable for oral or parenteral administration.

## Patentansprüche

**1.** Chlorin-e4-Natrium mit der Struktur:

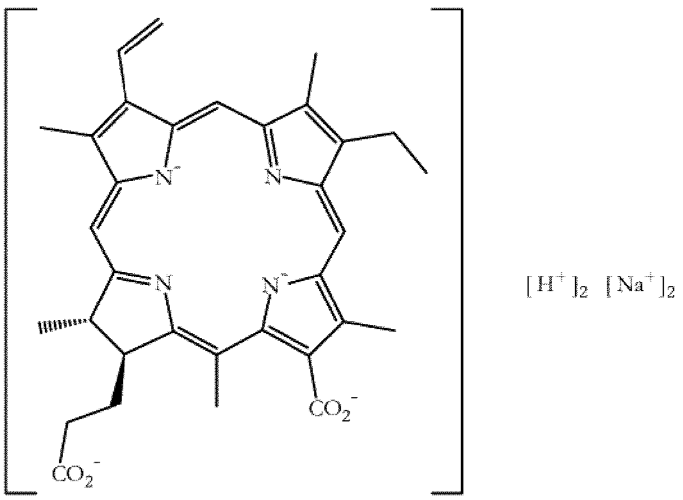

**2.** Chlorin-e4-Natrium nach Anspruch 1 mit der Struktur:

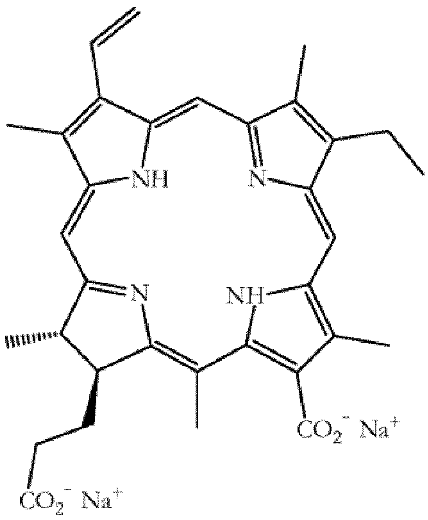

3. Verfahren zum Herstellen des Chlorin-e4-Natriums nach Anspruch 1 oder 2, umfassend den Schritt des Umsetzens von Chlorin e4 mit einer Natriumverbindung.

4. Verfahren nach Anspruch 3, wobei die Natriumverbindung Natriumhydroxid ist.

5. Verfahren nach Anspruch 3 oder 4, wobei Chlorin e4 mit der Natriumverbindung in Gegenwart einer Base, vorzugsweise einer Aminbase, vorzugsweise Thiaminpyrophosphat, umgesetzt wird.

6. Verfahren nach einem der Ansprüche 3 bis 5, wobei Chlorin e4, die Natriumverbindung und die optionale Base in einem Molverhältnis von 0,15-1 : 45-60 : 0-12 verwendet werden.

7. Chlorin-e4-Natrium nach Anspruch 1 oder 2 zur Verwendung in der Medizin.

8. Chlorin-e4-Natrium nach einem der Ansprüche 1, 2 oder 7 zur Verwendung in der fotodynamischen Therapie oder der Cytolumineszenztherapie.

9. Chlorin-e4-Natrium nach einem der Ansprüche 1, 2, 7 oder 8 zur Verwendung bei der Behandlung von:

(i) einer Krankheit, die durch gutartige oder bösartige zelluläre Hyperproliferation oder durch Bereiche mit Neovaskularisation gekennzeichnet ist; und/oder
(ii) einem gutartigen oder bösartigen Tumor; und/oder
(iii) Krebs im Frühstadium; zervikaler Dysplasie; Weichteilsarkom; einem Keimzelltumor; Retinoblastom; Lymphom; Hodgkin-Lymphom; Kopf- und Halskrebs; Oral- oder Mundkrebs; oder Krebs des Blutes, der Prostata, des Gebärmutterhalses, der Gebärmutter, der Scheide oder anderer weiblicher Adnexe, der Brust, des Nasopharynx, der Luftröhre, des Kehlkopfs, der Bronchien, der Bronchiolen, der Lunge, der Hohlorgane, der Speiseröhre, des Magens, des Gallengangs, des Darms, des Dickdarms, des Kolorektums, des Rektums, der Blase, des Harnleiters, der Niere, der Leber, der Gallenblase, der Milz, des Gehirns, des Lymphsystems, der Knochen, der Haut oder der Bauchspeicheldrüse.

10. Chlorin-e4-Natrium nach einem der Ansprüche 1, 2 oder 7 zur Verwendung in der fotodynamischen Diagnose.

11. Chlorin-e4-Natrium zur Verwendung nach einem der Ansprüche 7 bis 10, wobei das Chlorin-e4-Natrium zur Verabreichung vor einer Verabreichung von Bestrahlung geeignet ist, wobei die Bestrahlung vorzugsweise elektromagnetische Strahlung mit einer Wellenlänge im Bereich von 500 nm bis 1000 nm ist.

12. Pharmazeutische Zusammensetzung, umfassend das Chlorin-e4-Natrium nach einem der Ansprüche 1-2 oder 7-11 und einen pharmazeutisch verträglichen Träger oder ein pharmazeutisch verträgliches Verdünnungsmittel.

13. Pharmazeutische Zusammensetzung nach Anspruch 12 zur Verwendung in der fotodynamischen Therapie oder der Cytolumineszenztherapie.

**14.** Pharmazeutische Zusammensetzung nach Anspruch 12 oder 13 zur Verwendung bei der Behandlung von:

(i) einer Krankheit, die durch gutartige oder bösartige zelluläre Hyperproliferation oder durch Bereiche mit Neovaskularisation gekennzeichnet ist; und/oder
(ii) einem gutartigen oder bösartigen Tumor; und/oder
(iii) Krebs im Frühstadium; zervikaler Dysplasie; Weichteilsarkom; einem Keimzelltumor; Retinoblastom; Lymphom; Hodgkin-Lymphom; Kopf- und Halskrebs; Oral- oder Mundkrebs; oder Krebs des Blutes, der Prostata, des Gebärmutterhalses, der Gebärmutter, der Scheide oder anderer weiblicher Adnexe, der Brust, des Nasopharynx, der Luftröhre, des Kehlkopfs, der Bronchien, der Bronchiolen, der Lunge, der Hohlorgane, der Speiseröhre, des Magens, des Gallengangs, des Darms, des Dickdarms, des Kolorektums, des Rektums, der Blase, des Harnleiters, der Niere, der Leber, der Gallenblase, der Milz, des Gehirns, des Lymphsystems, der Knochen, der Haut oder der Bauchspeicheldrüse.

**15.** Pharmazeutische Zusammensetzung nach Anspruch 12 zur Verwendung in der fotodynamischen Diagnose.

**16.** Pharmazeutische Zusammensetzung nach einem der Ansprüche 12 bis 15, wobei die pharmazeutische Zusammensetzung zur Verabreichung vor einer Verabreichung von Bestrahlung geeignet ist, wobei die Bestrahlung vorzugsweise elektromagnetische Strahlung mit einer Wellenlänge im Bereich von 500 nm bis 1000 nm ist.

**17.** Pharmazeutische Zusammensetzung nach einem der Ansprüche 12 bis 16, wobei die pharmazeutische Zusammensetzung in einer Form vorliegt, die zur oralen, parenteralen (einschließlich intravenösen, subkutanen, intramuskulären, intradermalen, intratrachealen, intraperitonealen, intraartikulären, intraabdominalen, intrakranialen und epiduralen), transdermalen, Atemwegs- (Aerosol), rektalen, vaginalen oder topischen (einschließlich bukkalen, mukosalen und sublingualen) Verabreichung geeignet ist, wobei die pharmazeutische Zusammensetzung vorzugsweise in einer Form vorliegt, die zur oralen oder parenteralen Verabreichung geeignet ist.

**Revendications**

**1.** Chlorine e4 sodium, ayant la structure :

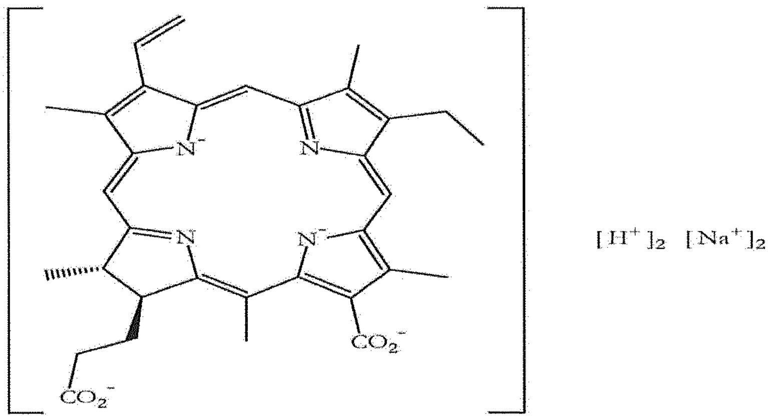

.

**2.** Chlorine e4 sodium selon la revendication 1, ayant la structure :

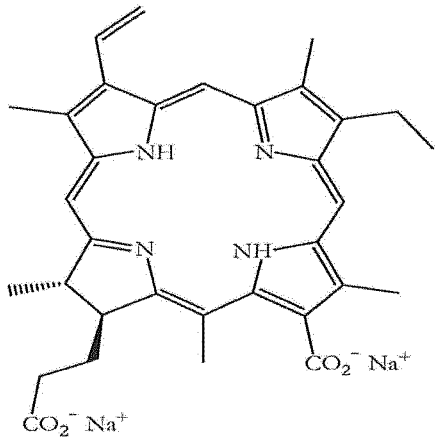

3. Procédé de préparation de la chlorine e4 sodium selon la revendication 1 ou 2, comprenant l'étape de réaction de la chlorine e4 avec un composé de sodium.

4. Procédé selon la revendication 3, dans lequel le composé de sodium est l'hydroxyde de sodium.

5. Procédé selon la revendication 3 ou 4, dans lequel la chlorine e4 est mise en réaction avec le composé de sodium en présence d'une base, de préférence une base amine, de préférence du pyrophosphate de thiamine.

6. Procédé selon l'une quelconque des revendications 3 à 5, dans lequel la chlorine e4, le composé de sodium et la base éventuelle sont utilisés dans un rapport molaire de 0,15-1:45-60:0-12.

7. Chlorine e4 sodium selon la revendication 1 ou 2, destinée à être utilisée en médecine.

8. Chlorine e4 sodium selon l'une quelconque des revendications 1, 2 ou 7, destinée à être utilisée en thérapie photodynamique ou en thérapie cytoluminescente.

9. Chlorine e4 sodium selon l'une quelconque des revendications 1, 2, 7 ou 8, destinée à être utilisée dans le traitement :

(i) d'une maladie **caractérisée par** une hyperprolifération cellulaire bénigne ou maligne ou par des zones de néovascularisation ; et/ou
(ii) d'une tumeur bénigne ou maligne ; et/ou
(iii) d'un cancer précoce ; d'une dysplasie cervicale ; d'un sarcome des tissus mous ; d'une tumeur des cellules germinales ; rétinoblastome ; d'un lymphome ; d'un lymphome de Hodgkin ; d'un cancer de la tête et du cou ; d'un cancer buccal ou de la bouche ; d'un cancer du sang, de la prostate, du col de l'utérus, de l'utérus, du vagin ou d'autres annexes féminines, du sein, du naso-pharynx, de la trachée, du larynx, des bronches, des bronchioles, du poumon, des organes creux, de l'œsophage, de l'estomac, des voies biliaires, de l'intestin, du côlon, du colorectum, du rectum, de la vessie, de l'uretère, des reins, du foie, de la vésicule biliaire, de la rate, du cerveau, du système lymphatique, des os, de la peau ou du pancréas.

10. Chlorine e4 sodium selon l'une quelconque des revendications 1, 2 ou 7, destinée à être utilisée en diagnostic photodynamique.

11. Chlorine e4 sodium destinée à être utilisée selon l'une quelconque des revendications 7 à 10, dans laquelle la chlorine e4 sodium est adaptée pour une administration avant l'administration d'irradiation, de préférence dans laquelle l'irradiation est un rayonnement électromagnétique avec une longueur d'onde comprise dans la plage allant de 500 nm à 1 000 nm.

12. Composition pharmaceutique comprenant la chlorine e4 sodium selon l'une quelconque des revendications 1 à 2 ou 7 à 11 et un support ou un diluant pharmaceutiquement acceptable.

13. Composition pharmaceutique selon la revendication 12, destinée à être utilisée en thérapie photodynamique ou en thérapie cytoluminescente.

**14.** Composition pharmaceutique selon la revendication 12 ou 13, destinée à être utilisée dans le traitement :

(i) d'une maladie **caractérisée par** une hyperprolifération cellulaire bénigne ou maligne ou par des zones de néovascularisation ; et/ou
(ii) d'une tumeur bénigne ou maligne ; et/ou
(iii) d'un cancer précoce ; d'une dysplasie cervicale ; d'un sarcome des tissus mous ; d'une tumeur des cellules germinales ; d'un rétinoblastome ; d'un lymphome ; d'un lymphome de Hodgkin ; d'un cancer de la tête et du cou ; d'un cancer buccal ou de la bouche ; d'un cancer du sang, de la prostate, du col de l'utérus, de l'utérus, du vagin ou d'autres annexes féminines, du sein, du naso-pharynx, de la trachée, du larynx, des bronches, des bronchioles, du poumon, des organes creux, de l'œsophage, de l'estomac, des voies biliaires, de l'intestin, du côlon, du colorectum, du rectum, de la vessie, de l'uretère, des reins, du foie, de la vésicule biliaire, de la rate, du cerveau, du système lymphatique, des os, de la peau ou du pancréas.

**15.** Composition pharmaceutique selon la revendication 12, destinée à être utilisée en diagnostic photodynamique.

**16.** Composition pharmaceutique selon l'une quelconque des revendications 12 à 15, dans laquelle la composition pharmaceutique est adaptée pour une administration avant l'administration d'irradiation, de préférence dans laquelle l'irradiation est un rayonnement électromagnétique avec une longueur d'onde comprise dans la plage allant de 500 nm à 1 000 nm.

**17.** Composition pharmaceutique selon l'une quelconque des revendications 12 à 16, dans laquelle la composition pharmaceutique est sous une forme appropriée pour une administration par voie orale, parentérale (y compris intraveineuse, sous-cutanée, intramusculaire, intradermique, intratrachéale, intrapéritonéale, intraarticulaire, intraabdominale, intracrânienne et épidurale), transdermique, aérienne (aérosol), rectale, vaginale ou topique (y compris buccale, muqueuse et sublinguale), de préférence dans laquelle la composition pharmaceutique est sous une forme appropriée pour une administration par voie orale ou parentérale.

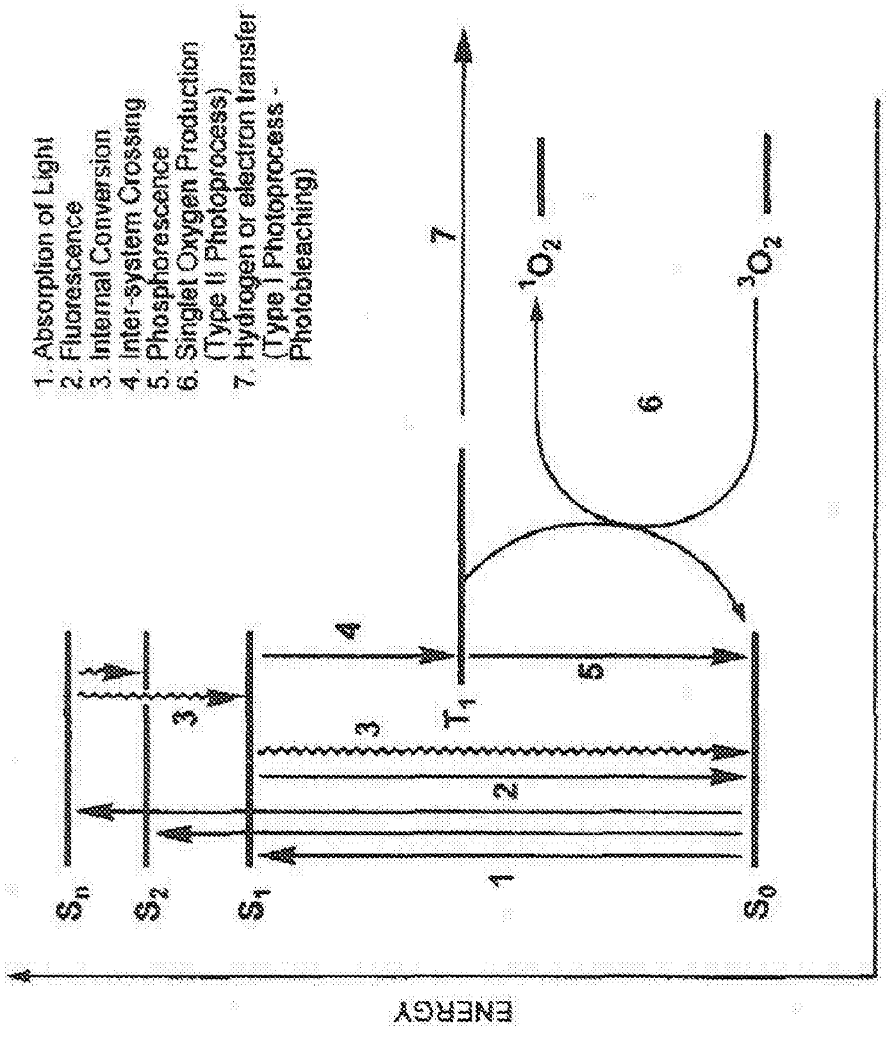
1. Absorption of Light
2. Fluorescence
3. Internal Conversion
4. Inter-system Crossing
5. Phosphorescence
6. Singlet Oxygen Production (Type II Photoprocess)
7. Hydrogen or electron transfer (Type I Photoprocess - Photobleaching)
$S_n$
$S_2$
$S_1$
$S_0$
$T_1$
$^1O_2$
$^3O_2$
1
2
3
3
4
5
6
7
ENERGY

Figure 1

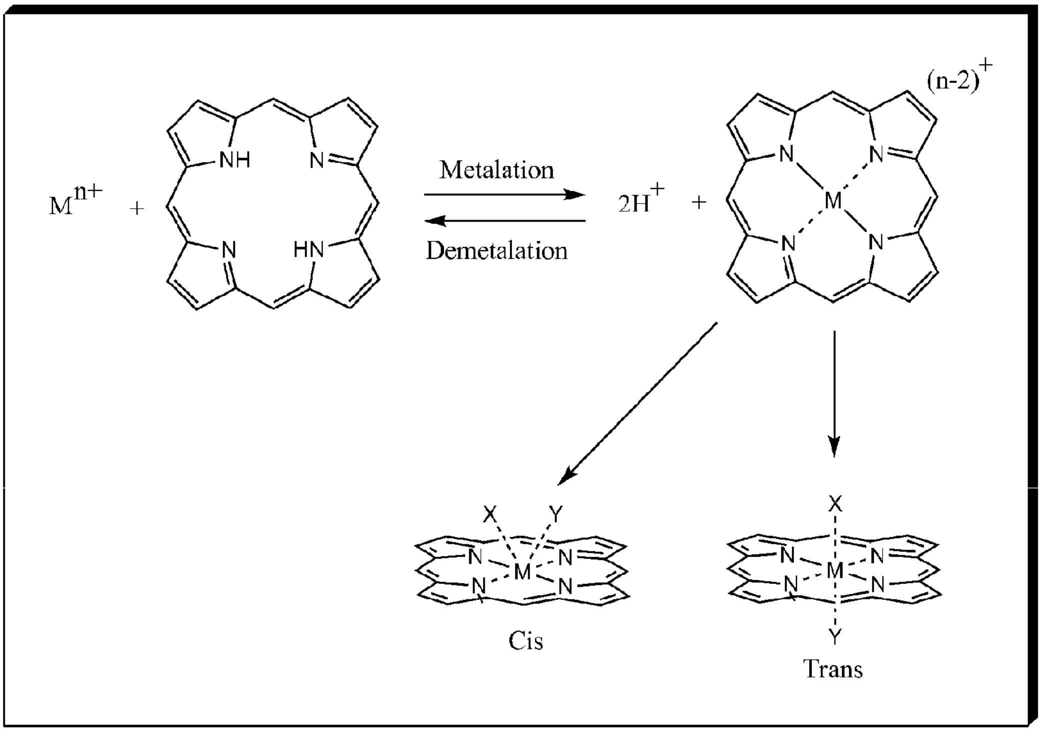


Figure 2

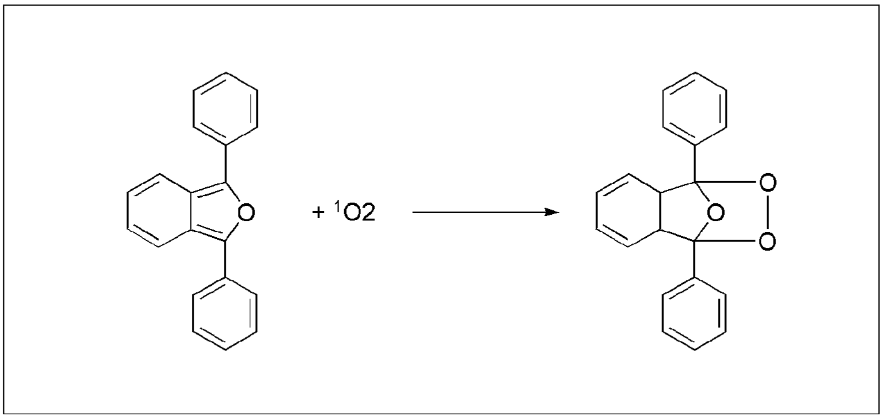

Figure 3

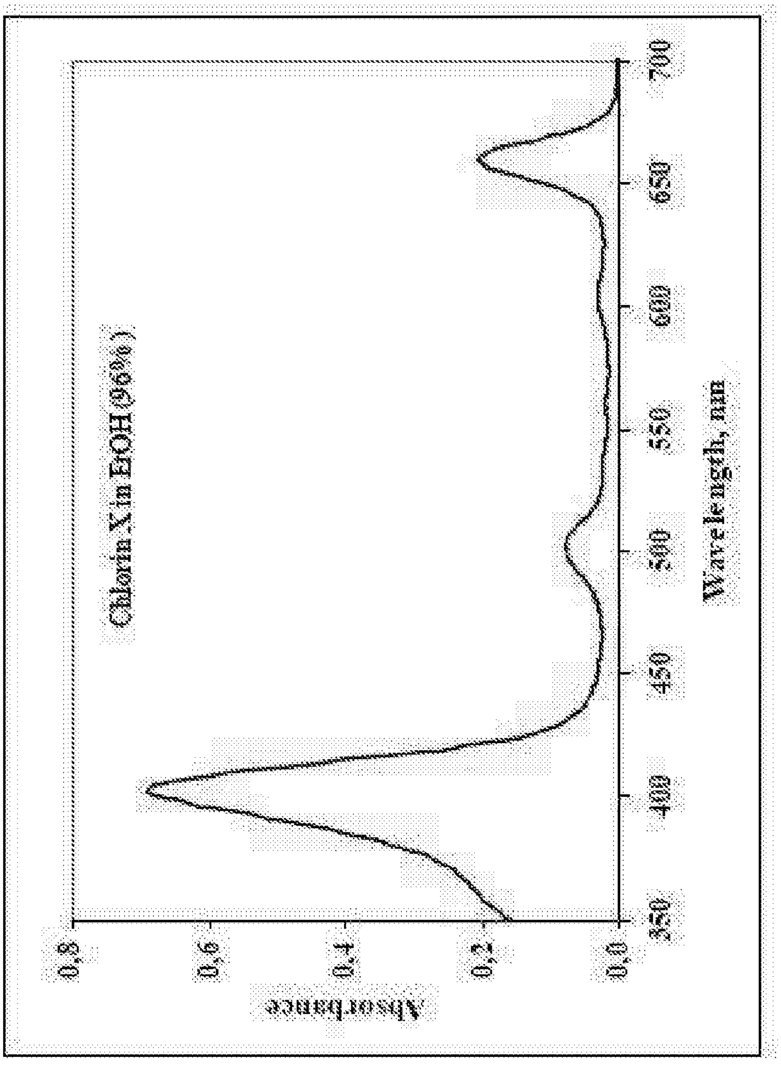
Chlorin X in EtOH (96%)
Absorbance
0,0
0,2
0,4
0,6
0,8
350
400
450
500
550
600
650
700
Wavelength, nm

Figure 4

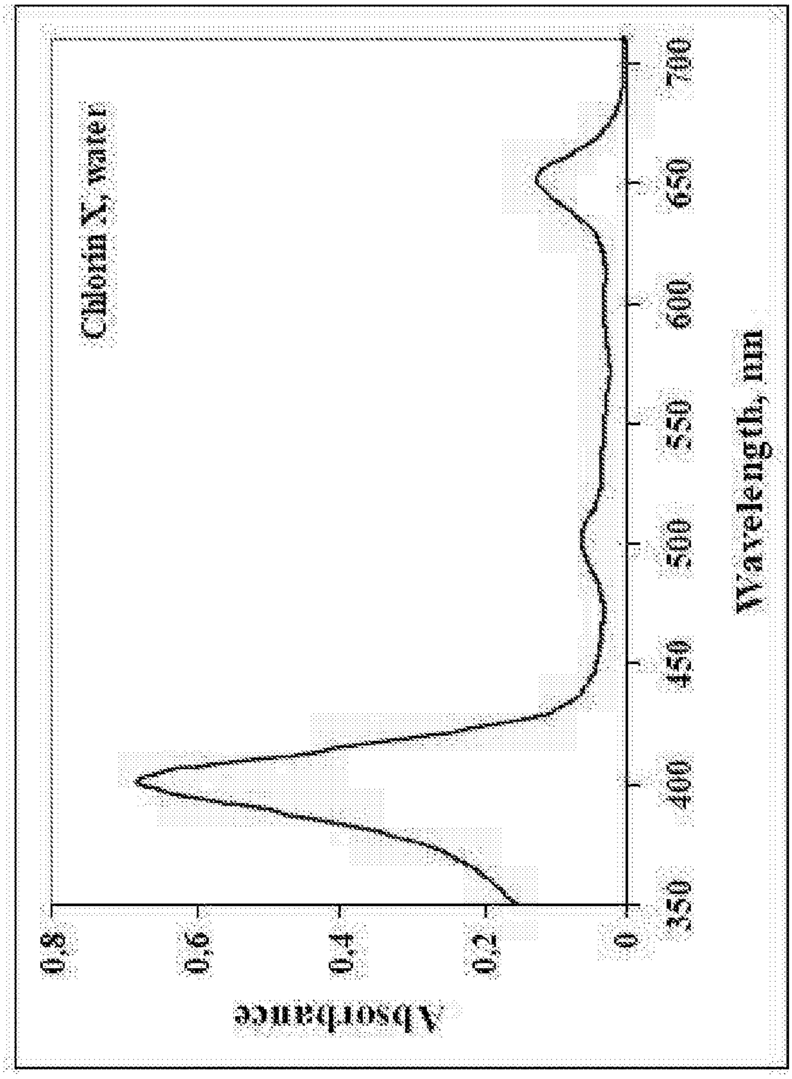
Chlorin X, water
Absorbance
0,8
0,6
0,4
0,2
0
350
400
450
500
550
600
650
700
Wavelength, nm

Figure 5

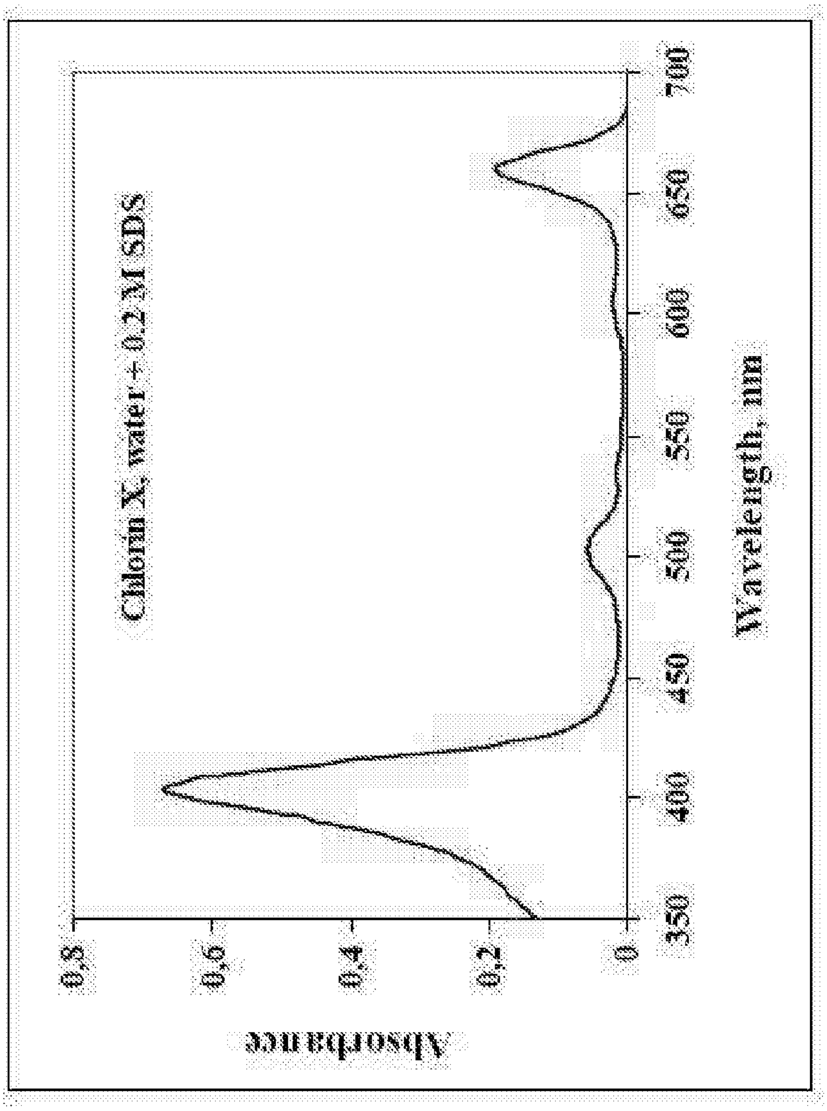
Chlorin X, water + 0.2 M SDS
Absorbance
Wavelength, nm
0
0.2
0.4
0.6
0.8
350
400
450
500
550
600
650
700

Figure 6

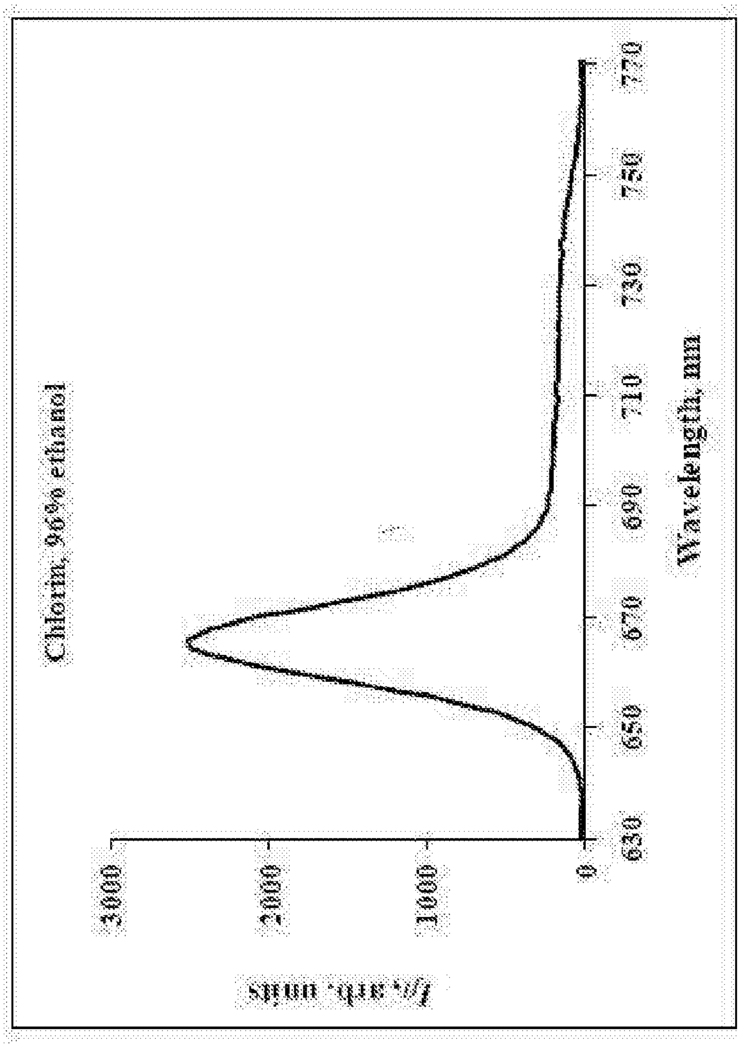
Chlorin, 96% ethanol
3000
2000
1000
0
630
650
670
690
710
730
750
770
Wavelength, nm

Figure 7

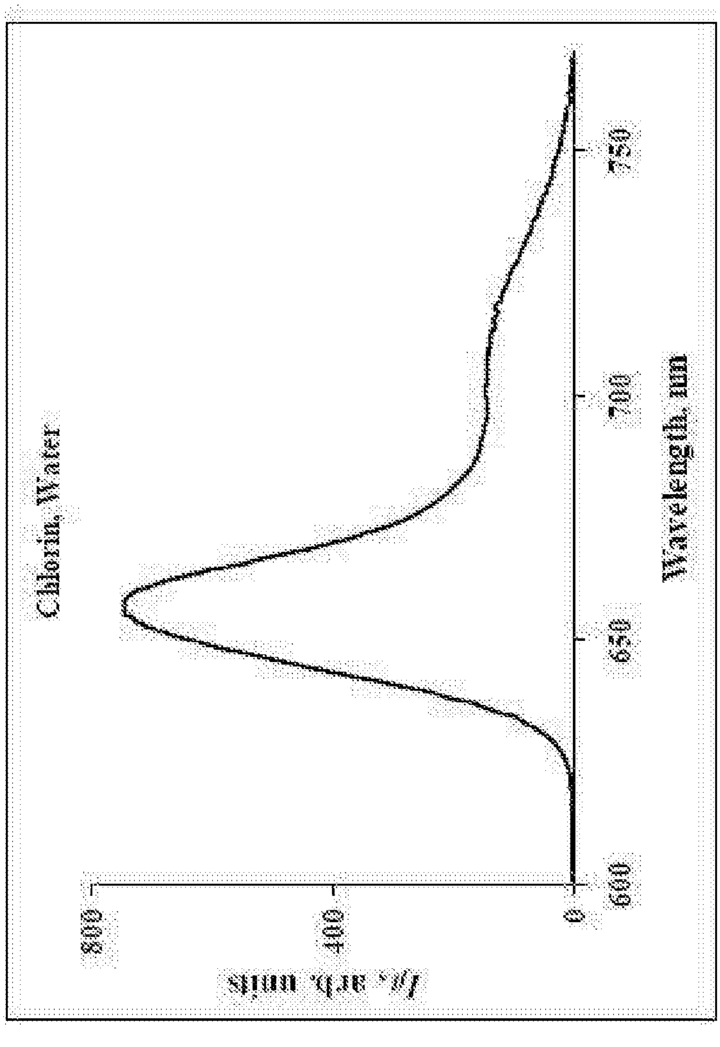
Chlorin, Water
800
400
0
600
650
700
750
Wavelength, nm
arb. units

Figure 8

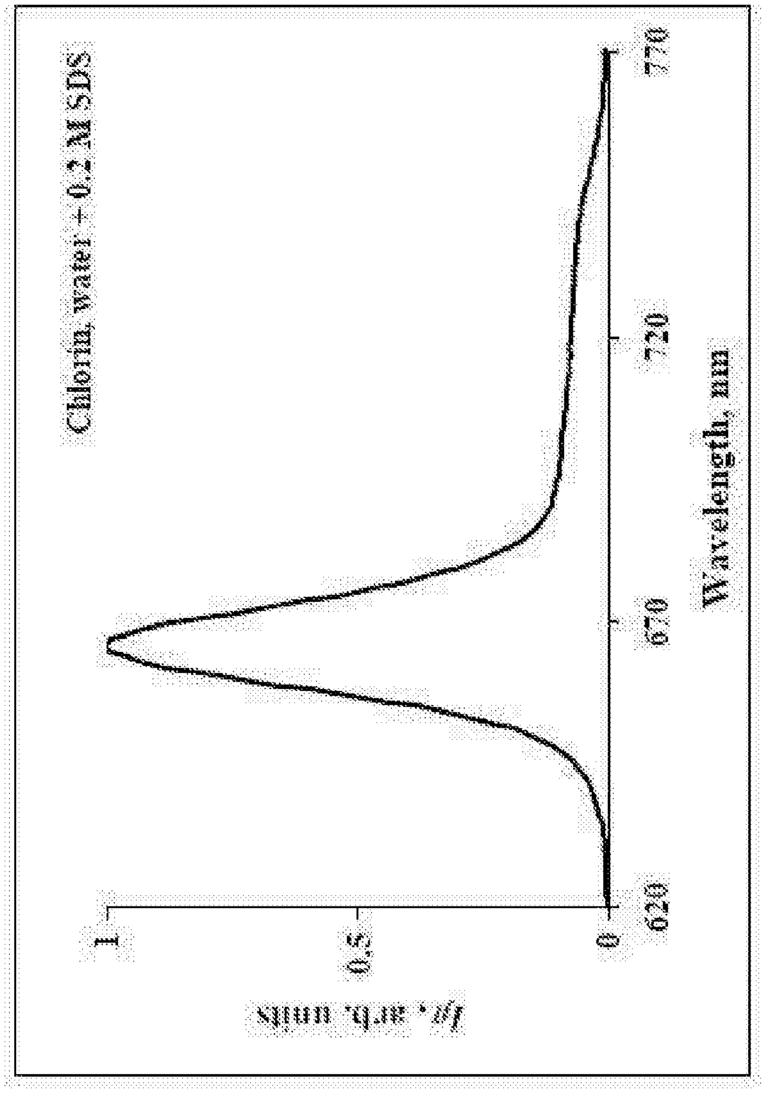

Chlorin, water + 0.2 M SDS
Ifl, arb. units
1
0.5
0
620
670
720
770
Wavelength, nm


Figure 9

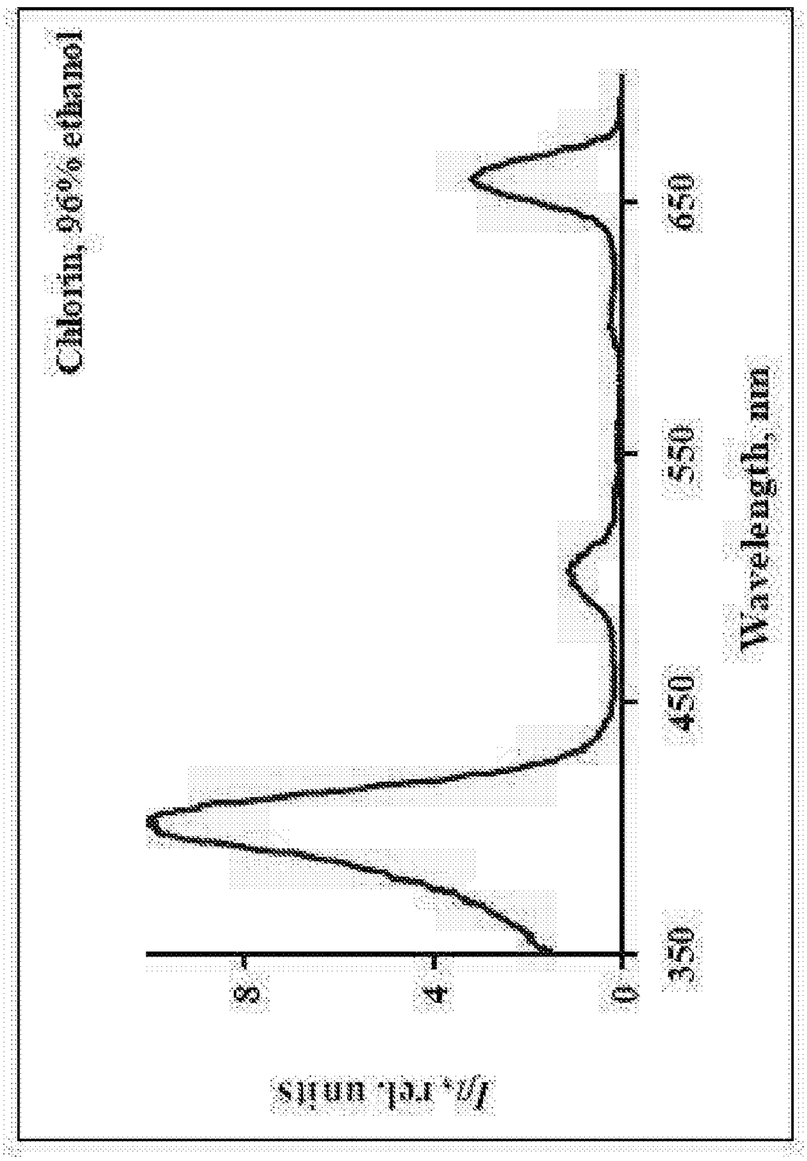
Chlorin, 96% ethanol
$I_{fl}$, rel. units
8
4
0
350
450
550
650
Wavelength, nm

Figure 10

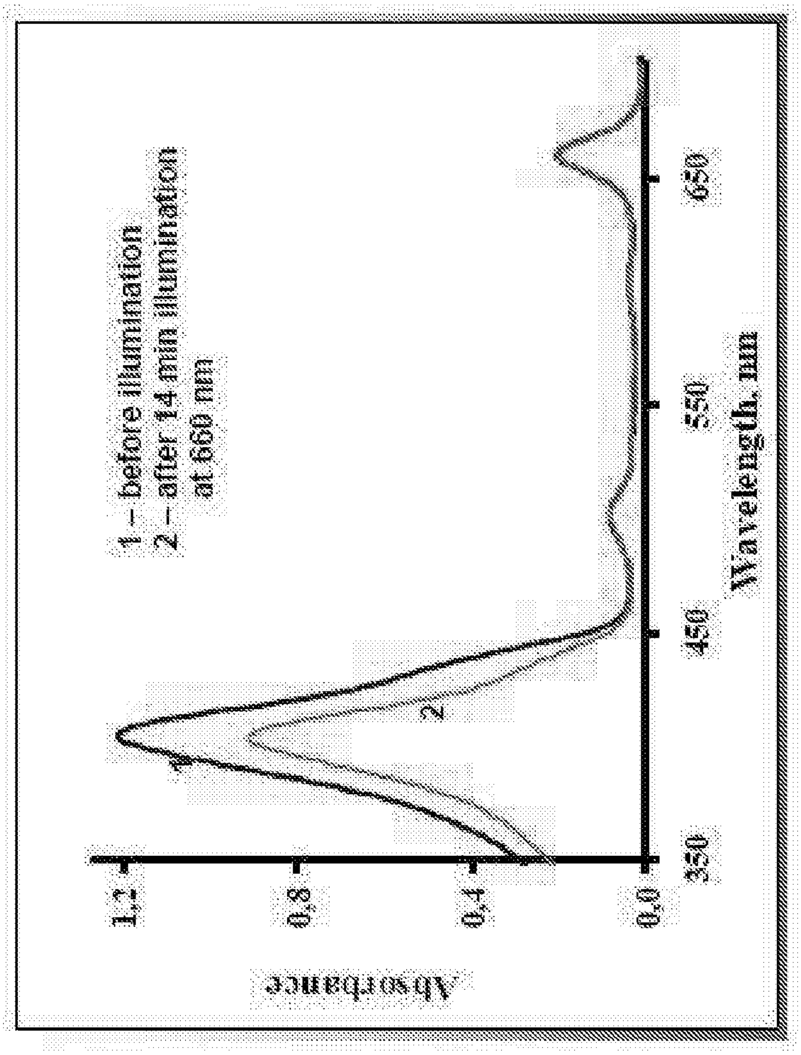
1 – before illumination
2 – after 14 min illumination
at 660 nm
Absorbance
Wavelength, nm
1,2
0,8
0,4
0,0
350
450
550
650
1
2

Figure 11

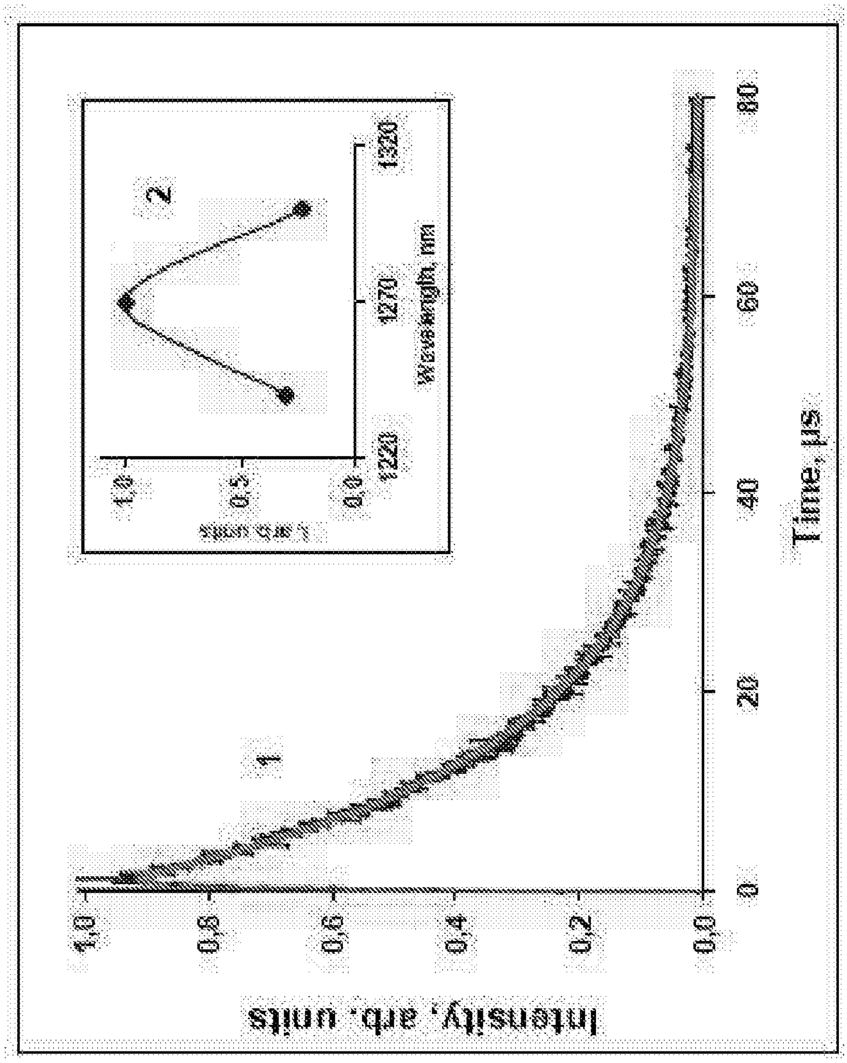
Intensity, arb. units
1,0
0,8
0,6
0,4
0,2
0,0
0
20
40
60
80
Time, µs
1
2
1,0
0,5
0,0
1220
1270
1320
Wavelength, nm

Figure 12

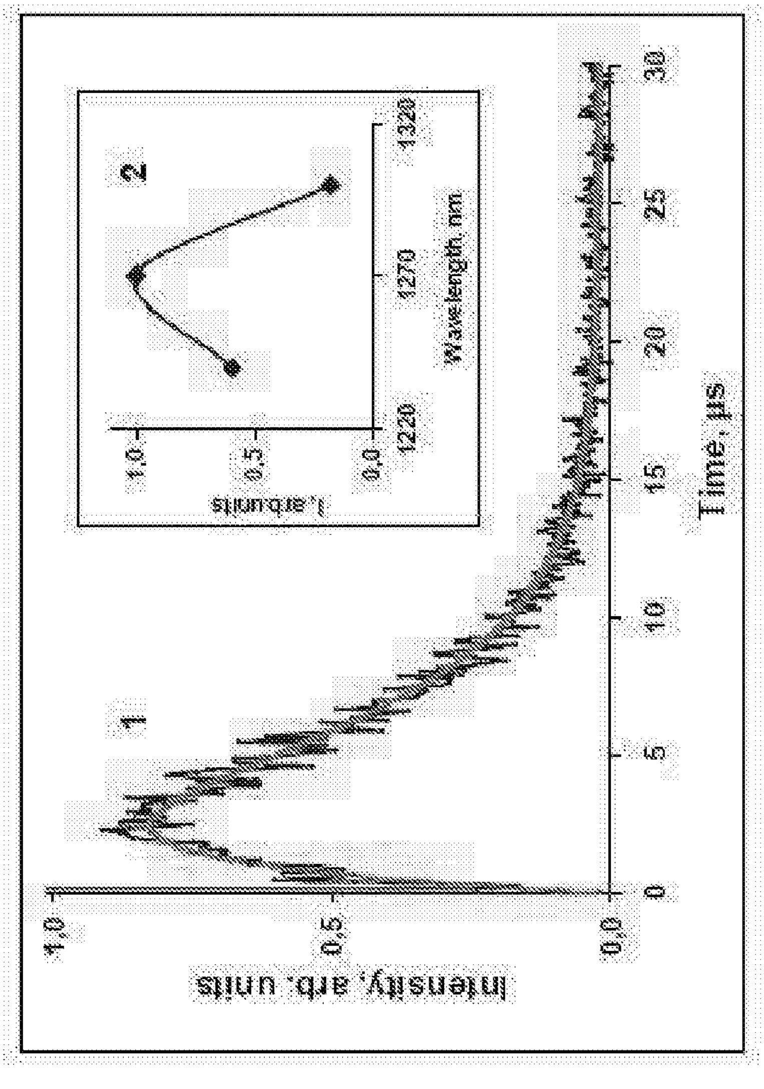
Intensity, arb. units
1,0
0,5
0,0
0
5
10
15
20
25
30
Time, µs
1
2
I, arb.units
1,0
0,5
0,0
1220
1270
1320
Wavelength, nm

Figure 13

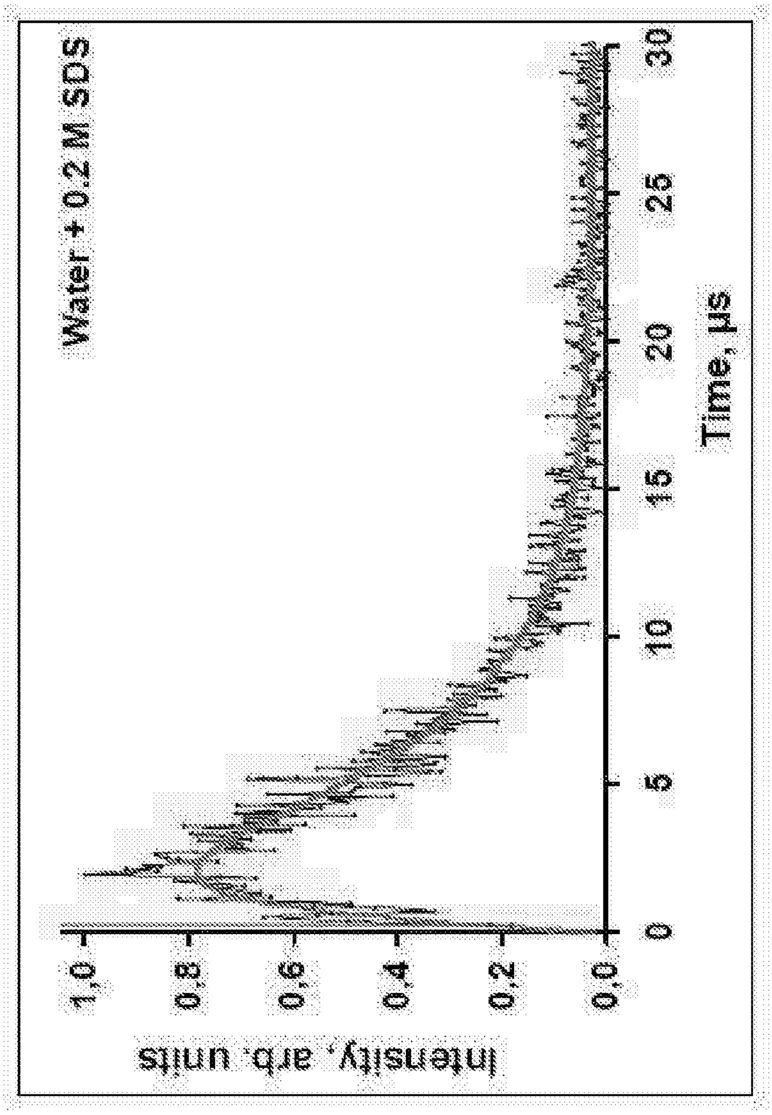
Water + 0.2 M SDS
Intensity, arb. units
1,0
0,8
0,6
0,4
0,2
0,0
0
5
10
15
20
25
30
Time, µs

Figure 14

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009040411 A


### Non-patent literature cited in the description


- **WONG et al.** *The Journal of Pharmacology and Experimental Therapeutics,* 1967, vol. 155 (1), 50-57
- **LI et al.** *Oncology Reports,* 2012, vol. 27, 1455-1460
- **K. KALYANASUNDARAM ; M. NEUMANN-SPALLART.** Photophysical and redox properties of water soluble porphyrins in aqueous media. *J. Phys. Chem.,* 1982, vol. 86, 5163-5169
- **A.T. GRADYUSHKO ; A.N. SEVCHENKO ; K.N. SOLOVYOV ; M.P. TSVIRKO.** Energetics of photophysical processes in chlorophyll-like molecules. *Photochem. Photobiol.,* 1970, vol. 11, 387-400
- **A.A. KRASNOVSKY JR.** Photodynamic action and singlet oxygen. *Biofizika,* 2004, vol. 49, 305-321
- Singlet oxygen and primary mechanisms of photodynamic therapy and photodynamic diseases. **A.A. KRASNOVSKY JR.** Photodynamic therapy at the cellular level. 2007, 17-62
- **D.A. BUTORINA ; A.A. KRASNOVSKY JR. ; A.V. PRIEZZEV.** Investigation of the kinetic parameters of singlet molecular oxygen in aqueous porphyrin solutions, Influence of detergents and the quencher sodium azide. *Biofizika,* 2003, vol. 48, 189-196
- **A.A. KRASNOVSKY JR.** Luminescence and photochemical studies of singlet oxygen photonics. *J. Photochem. Photobiol. A: Chem.,* 2008, vol. 196, 210-218
- **N. KUZNETSOVA ; D. MAKAROV ; O. YUZHAKOVA ; A. STRIZHAKOV ; Y. ROUMBAL ; L. ULANOVA ; A. KRASNOVSKY ; O. KALIYA.** Photophysical properties and photodynamic activity of octacationic oxotitanium(IV) phthalocyanines. *Photochemical Photobiological Sciences,* 2009, vol. 8, 1724-1733
- **F. WILKINSON ; W.P. HELMAN ; A.B. ROSS.** Quantum yields of the photosensitized formation of the lowest electronically excited singlet states of molecular oxygen. *J. Phys. Chem. Ref. Data,* 1993, vol. 22 (2), 113-262
- **C. TANIELIAN ; C. WOLF ; M. ESCH.** Singlet oxygen production in water: Aggregation and charge transfer effects. *J. Phys. Chem.,* 1996, vol. 100, 6555-6560
- **A.A. KRASNOVSKY JR. ; Y.V. ROUMBAL ; A.A. STRIZHAKOV.** Rates of O2 production upon direct excitation of molecular oxygen by 1270 nm laser radiation in air-saturated alcohols and micellar aqueous dispersions. *Chem. Phys. Lett.,* 2008, vol. 458, 195-199